(19) Europäisches Patentamt
European Patent Office
Office européen des brevets

(11) **EP 1 238 585 B1**

(12) **EUROPÄISCHE PATENTSCHRIFT**

(45) Veröffentlichungstag und Bekanntmachung des Hinweises auf die Patenterteilung:
**11.05.2005 Patentblatt 2005/19**

(51) Int Cl.$^7$: **A01N 37/12**, A01N 37/20

(21) Anmeldenummer: **02004824.5**

(22) Anmeldetag: **02.03.2002**

(54) **Verwendung von Esterquats**

Use of esterquats

Utilisation d'esters quaternaires

(84) Benannte Vertragsstaaten:
**DE ES FR GB IT**

(30) Priorität: **10.03.2001 DE 10111559**

(43) Veröffentlichungstag der Anmeldung:
**11.09.2002 Patentblatt 2002/37**

(73) Patentinhaber: **Cognis Iberia, S.L.**
**08755 Castellbisbal, (Barcelona) (ES)**

(72) Erfinder:
- **Bigorra Llosas, Joaquin, Dr.**
  **08203 Sabadell (ES)**
- **Bonastre Gilabert, Nuria, Dr.**
  **08210 Barbera del Vall (ES)**
- **Pi Subirana, Rafael Dr.**
  **08400 Granollers (ES)**

(74) Vertreter: **Fabry, Bernd, Dr.**
**Cognis Deutschland GmbH & Co. KG**
**Postfach 13 01 64**
**40551 Düsseldorf (DE)**

(56) Entgegenhaltungen:
**EP-A- 0 770 594** **DE-A- 19 742 222**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

### Gebiet der Erfindung

Die Erfindung befindet sich auf dem Gebiet der bioziden Wirkstoffe für den Einsatz im Bereich der Detergentien und Kosmetik und betrifft die Verwendung spezieller kationischer Tenside.

### Stand der Technik

Mikrobizide Wirkstoffe werden in der Regel eingesetzt, um Mikroorganismen an ihrer Vermehrung zu hindern oder ganz abzutöten. Bislang haben sich als mikrobizide Wirkstoffe quaternäre Ammoniumverbindungen wie Benzalkoniumchlorid oder Cetyltrimethylammoniumchlorid gut bewährt. Es hat sich jedoch gezeigt, dass derartige Verbindungen in der Kläranlage nur schwer biologisch abbaubar sind. Es besteht demnach ein Bedürfnis nach mikrobizid wirkenden Verbindungen, die in ihrer Leistungsfähigkeit mit den quaternären Ammoniumverbindungen vergleichbar sind, aber über eine deutlich verbesserte biologische Abbaubarkeit verfügen.

Aus der amerikanischen Patentschrift **US 3,910,971** sind quaternäre Carbonsäurealkanolaminestersalze als mikrobizide Wirkstoffe bekannt, die durch Umsetzung von 2-Dimethylamino-2-methyl-1-propanol mit Fettsäuren und anschließender Quatemierung hergestellt werden. Diese Verbindungen zeigen jedoch gegenüber dem gramnegativen Bakterium Pseudomonas aeruginosa kaum Wirksamkeit, was angesichts des häufig auftretenden Wasserkeimes, der insbesondere zu Produktverkeimungen führt, von Nachteil ist. In der europäischen Patentanmeldung **EP 0461419 A1** werden Poly(oxyalkylen)aminoalkanolester sowie deren Ammoniumverbindungen beschrieben, die ebenfalls eine mikrobizide Wirkung aufweisen sollen. Diese Verbindungen weisen jedoch zwingend mindestens einen Hydroxyisopropylrest auf und tragen an dem Stickstoffatom eine Methylgruppe. Über die Wirksamkeit dieser Verbindungen gegenüber Bakterien, insbesondere gegenüber dem häufig vorkommenden Bakterium Pseudomonas aeruginosa, werden keine Angaben gemacht.

Gegenstand der deutschen Patentanmeldung **DE 19742222 A1** sind Esterquats auf Basis kurzkettiger Carbonsäuren und deren Verwendung als Biozide. Diese Stoffe stellen jedoch nur bedingt einen zufriedenstellenden Kompromiss zwischen ausreichender Wirksamkeit und erforderlicher biologischer Abbaubarkeit dar. Des weiteren sei auf die Europäischen Patentschrift **EP 0770594** B1 (Cognis) verwiesen, aus der Esterquats auf Basis von Mono- und Dicarbonsäuren bekannt sind.

Die komplexe Aufgabe der vorliegenden Erfindung hat somit darin bestanden, neue Biozide zur Verfügung zu stellen, die frei von den geschilderten Nachteilen sind und dabei gleichzeitig über eine bessere biologische Abbaubarkeit und eine höhere Wirksamkeit gegenüber Mikroorganismen verfügen.

### Beschreibung der Erfindung

Gegenstand der Erfindung ist die Verwendung von Esterquats als Biozide, welche sich dadurch auszeichnen, dass sich die Acylkomponente der Esterquats von Gemischen von

(a) Monocarbonsäuren und
(b) Dicarbonsäuren

ableitet.

Überraschenderweise wurde gefunden, dass der Einbau auch schon sehr geringer Mengen von Dicarbonsäuren in die Acylkomponente der Dicarbonsäuren nicht nur zu einer verbesserten biologischen Abbaubarkeit als auch zu einer höheren Wirksamkeit gegenüber Mikroorganismen führt, obschon diese Effekte in aller Regel gerade gegenläufig sind. Dies trifft insbesondere für solche Esterquats zu, die sich von kurzkettigen Fettsäuren mit 6 bis 12 und vorzugsweise 8 bis 10 Kohlenstoffatomen, besonders bevorzugt in Abmischung mit Adipinsäure ableiten.

#### Esterquats

Unter der Bezeichnung "Esterquats" werden im allgemeinen quaternierte Fettsäuretriethanolaminestersalze verstanden. Es handelt sich dabei um bekannte Stoffe, die man nach den einschlägigen Methoden der präparativen organischen Chemie erhalten kann. In diesem Zusammenhang sei auf die Internationale Patentanmeldung **WO 91/01295** (Henkel) verwiesen, nach der man Triethanolamin in Gegenwart von unterphosphoriger Säure mit Fettsäuren partiell verestert, Luft durchleitet und anschließend mit Dimethylsulfat oder Ethylenoxid quaterniert. Aus der Deutschen Patentschrift **DE 4308794 C1** (Henkel) ist überdies ein Verfahren zur Herstellung fester Esterquats bekannt, bei dem man die Quaternierung von Triethanolaminestern in Gegenwart von geeigneten Dispergatoren, vorzugsweise Fettalkoholen, durchführt. Übersichten zu diesem Thema sind beispielsweise von R.Puchta et al. in **Tens.Surf.Det., 30, 186 (1993),** M.Brock in **Tens.Surf.Det. 30, 394 (1993),** R.Lagerman et al. in **J.Am. Oil.Chem.Soc., 71, 97 (1994)** sowie I. Shapiro in **Cosm.Toil. 109, 77 (1994)** erschienen.

Typische Beispiele für Esterquats, die im Sinne der Erfindung Verwendung finden können, sind Produkte, deren Acylkomponente (a) sich von Monocarbonsäuren der Formel **(I)** ableiten,

$$R^1CO\text{-}OH \quad \textbf{(I)}$$

in der $R^1CO$ für einen linearen oder verzweigten, gesättigten oder ungesättigten, gegebenenfalls hydroxysubstituierten Acylrest mit 6 bis 22 Kohlenstoffatomen steht. Beispiele hierfür sind Capronsäure, Caprylsäure, Caprinsäure, Laurinsäure, Myristinsäure, Palmitinsäure, Isostearinsäure, Stearinsäure, Ölsäure, Elaidinsäure, Arachinsäure, Behensäure und Erucasäure sowie deren technische Mischungen, wie sie beispielsweise bei der Druckspaltung natürlicher Fette und Öle anfallen. Vorzugsweise werden solche Esterquats verwendet, deren Acylkomponente (a) sich von Monocarbonsäuren der Formel **(I)** ableitet, in der $R^1CO$ für einen linearen, gesättigten Acylrest mit 6 bis 12 und insbesondere 8 bis 10 Kohlenstoffatomen steht.

Des weiteren werden solche Esterquats eingesetzt, deren Acylkomponente (b) sich von Dicarbonsäuren der Formel **(II)** ableitet,

$$HOOC(CH_2)_nCOOH \quad \textbf{(II)}$$

in der n für Zahlen von 1 bis 10 steht. Beispiele hierfür sind Malonsäure, Bernsteinsäure, Maleinsäure, Fumarsäure, Glutarsäure, Sorbinsäure, Pimelinsäure, Azelainsäure, Sebacinsäure und/oder Dodecandisäure, aber vorzugsweise Adipinsäure. In Summe bevorzugt werden solche Esterquats eingesetzt, deren Acylkomponente (a) sich von Monocarbonsäuren der Formel **(I)** ableitet, in der $R^1CO$ für einen linearen, gesättigten Acylrest mit 6 bis 22 Kohlenstoffatomen und deren Acylkomponente (b) von der Adipinsäure ableitet. Das molare Verhältnis der Komponenten (a) und (b) kann dabei im Bereich von 1 : 99 bis 99 : 1, vorzugsweise 50 : 50 bis 90 : 10 und insbesondere 70 : 30 bis 80 : 20 betragen.

In einer speziellen Ausführungsform der Erfindung können solche Esterquats zum Einsatz gelangen, die quaternierte Fettsäuretriethanolaminestersalze der Formel **(III)** darstellen,

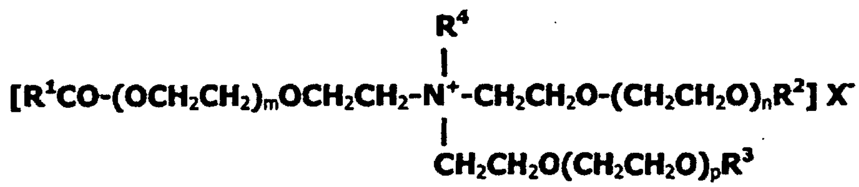

**(III)**

in der $R^1CO$ für Gemische von Mono- und Dicarbonsäuren, $R^2$ und $R^3$ unabhängig voneinander für Wasserstoff oder $R^1CO$, $R^4$ für einen Alkylrest mit 1 bis 4 Kohlenstoffatomen oder eine $(CH_2CH_2O)_qH$-Gruppe, m, n und p in Summe für 0 oder Zahlen von 1 bis 12, q für Zahlen von 1 bis 12 und X für Halogenid, Alkylsulfat oder Alkylphosphat steht.

Neben den quaternierten Fettsäuretriethanolaminestersalzen kommen als Esterquats ferner auch quaternierte Estersalze von Mono-/Dicarbonsäuregemischen mit Diethanolalkylaminen der Formel **(IV)** in Betracht,

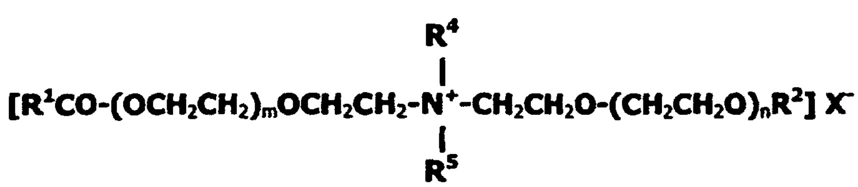

**(IV)**

in der $R^1CO$ für Gemische von Mono- und Dicarbonsäuren, $R^2$ für Wasserstoff oder $R^1CO$, $R^4$ und $R^5$ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

Als weitere Gruppe geeigneter Esterquats sind die quaternierten Estersalze von Mono/Dicarbonsäuregemischen mit 1,2-Dihydroxypropyldialkylaminen der Formel **(V)** zu nennen,

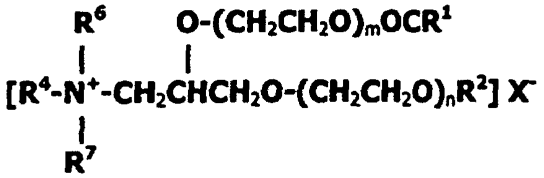

**(V)**

in der $R^1CO$ für Gemische von Mono- und Dicarbonsäuren, $R^2$ für Wasserstoff oder $R^1CO$, $R^4$, $R^6$ und $R^7$ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

Hinsichtlich der Auswahl der bevorzugten Fettsäuren und des optimalen Veresterungsgrades gelten die für **(III)** genannten Beispiele auch für die Esterquats der Formeln **(IV) und (V).**

Zur Herstellung der Esterquats der Formeln **(III)** bis **(V)** kann sowohl von Fettsäuren als auch den entsprechenden Triglyceriden in Abmischung mit den entsprechenden Dicarbonsäuren ausgegangen werden. Ein solches Verfahren, das stellvertretend für den entsprechenden Stand der Technik genannt werden soll, wird in der europäischen Patentschrift **EP 0750606 B1** (Cognis) vorgeschlagen.

Zur Herstellung der quaternierten Ester können die Gemische aus Mono- und Dicarbonsäuren und das Triethanolamin - bezogen auf die verfügbaren Carboxylfunktionen - im molaren Verhältnis von 1,1 : 1 bis 3 : 1 eingesetzt werden. Im Hinblick auf die anwendungstechnischen Eigenschaften der Esterquats hat sich ein Einsatzverhältnis von 1,2 : 1 bis 2,2 : 1, vorzugsweise 1,5 : 1 bis 1,9 : 1 als besonders vorteilhaft erwiesen. Die bevorzugten Esterquats stellen technische Mischungen von Mono-, Di- und Triestem mit einem durchschnittlichen Veresterungsgrad von 1,5 bis 1,9 dar

## Gewerbliche Anwendbarkeit

Ein weiterer Gegenstand der vorliegenden Erfindung betrifft die Verwendung oberflächenaktiver Zubereitungen mit bioziden Eigenschaften, die sich dadurch auszeichnen, dass sie eine wirksame Mengen von Estrerquats enthalten, deren Acylkomponente sich von Mischungen von

(a) Monocarbonsäuren und
(b) Dicarbonsäuren

ableitet. Unter wirksamen Mengen werden dabei Anteile von 0,1 bis 10, vorzugsweise 0,5 bis 8 und insbesondere 1 bis 5 Gew.-% - bezogen auf die Mittel - der genannten Esterquats verstanden.

## Wasch-, Spül-, Reinigungs- und Avivagemittel

Die oberflächenaktiven Mittel können beispielsweise Wasch-, Spül-, Reinigungs- oder Avivagemittel darstellen, die weiterhin für diese Zubereitungen typische Hilfs- und Zusatzstoffe enthalten können. Hierzu zählen beispielsweise anionische, nichtionische, kationische, amphotere oder zwitterionische Tenside, Builder, Co-Builder, öl- und fettlösende Stoffe, Bleichmittel, Bleichaktivatoren, Vergrauungsinhibitoren, Enzyme, Enzymstabilisatoren, Optische Aufheller, Polymere, Entschäumer, Sprengmittel, Duftstoffe, anorganische Salze und dergleichen, wie sie im folgenden näher erläutert werden.

### Anionische Tenside

Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenveneilung aufweisen. Vorzugsweise werden Alkylbenzolsulfonate, Alkylsulfate, Seifen, Alkansulfonate, Olefinsulfonate, Methylestersulfonate sowie deren Gemische eingesetzt. Typische Beispiele für nichtionische Tenside sind Fettalkoholpolyglycolether, Alkylphenolpolyglycolether, Fettsäurepolyglycolester, Fettsäureamidpolygly-colether, Fettaminpolyglycolether, alkoxylierte Triglyceride, Mischether bzw. Mischformale, Alk(en)yloligoglykoside, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Vorzugsweise werden Fettalkoholpolyglycolether, alkoxylierte Fettsäureniedrigalkylester oder Alkyloligoglucoside eingesetzt.

Builder

Die erfindungsgemäßen Wasch-, Spül-, Reinigungs- und Avivagemittel können des weiteren zusätzliche anorganische und organische Buildersubstanzen beispielsweise in Mengen von 10 bis 50 und vorzugsweise 15 bis 35 Gew.-% - bezogen auf die Mittel - enthalten, wobei als anorganische Buildersubstanzen hauptsächlich Zeolithe kristalline Schichtsilikate, amorphe Silikate und - soweit zulässig - auch Phosphate, wie z.B. Tripolyphosphat zum Einsatz kommen. Die Menge an Co-Builder ist dabei auf die bevorzugten Mengen an Phosphaten anzurechnen.

Der als Waschmittelbuilder häufig eingesetzte feinkristalline, synthetische und gebundenes Wasser enthaltende Zeolith ist vorzugsweise Zeolith A und/oder P. Als Zeolith P wird beispielsweise Zeolith MAP$^{(R)}$ (Handelsprodukt der Firma Crosfield) besonders bevorzugt. Geeignet sind jedoch auch Zeolith X sowie Mischungen aus A, X und/oder P wie auch Y. Von besonderem Interesse ist auch ein cokristallisiertes Natrium/Kalium-Aluminiumsilikat aus Zeolith A und Zeolith X, welches als VEGOBOND AX® (Handelsprodukt der Firma Condea Augusta S.p.A.) im Handel erhältlich ist. Der Zeolith kann als sprühgetrocknetes Pulver oder auch als ungetrocknete, von ihrer Herstellung noch feuchte, stabilisierte Suspension zum Einsatz kommen. Für den Fall, daß der Zeolith als Suspension eingesetzt wird, kann diese geringe Zusätze an nichtionischen Tensiden als Stabilisatoren enthalten, beispielsweise 1 bis 3 Gew.-%, bezogen auf Zeolith, an ethoxylierten $C_{12}$-$C_{18}$-Fettalkoholen mit 2 bis 5 Ethylenoxidgruppen, $C_{12}$-$C_{14}$-Fettalkoholen mit 4 bis 5 Ethylenoxidgruppen oder ethoxylierten Isotridecanolen. Geeignete Zeolithe weisen eine mittlere Teilchengröße von weniger als 10 μm (Volumenverteilung; Meßmethode: Coulter Counter) auf und enthalten vorzugsweise 18 bis 22 Gew.-%, insbesondere 20 bis 22 Gew.-% an gebundenem Wasser.

Geeignete Substitute bzw. Teilsubstitute für Phosphate und Zeolithe sind kristalline, schichtförmige Natriumsilikate der allgemeinen Formel $NaMSi_xO_{2x+1} \cdot yH_2O$, wobei M Natrium oder Wasserstoff bedeutet, x eine Zahl von 1,9 bis 4 und y eine Zahl von 0 bis 20 ist und bevorzugte Werte für x 2, 3 oder 4 sind. Derartige kristalline Schichtsilikate werden beispielsweise in der europäischen Patentanmeldung **EP 0164514 A1** beschrieben. Bevorzugte kristalline Schichtsilikate der angegebenen Formel sind solche, in denen M für Natrium steht und x die Werte 2 oder 3 annimmt. Insbesondere sind sowohl β- als auch δ-Natriumdisilikate $Na_2Si_2O_5 \cdot yH_2O$ bevorzugt, wobei β-Natriumdisilikat beispielsweise nach dem Verfahren erhalten werden kann, das in der internationalen Patentanmeldung **WO 91/08171** beschrieben ist. Weitere geeignete Schichtsilikate sind beispielsweise aus den Patentanmeldungen **DE 2334899 A1, EP 0026529 A1** und **DE 3526405 A1** bekannt. Ihre Verwendbarkeit ist nicht auf eine spezielle Zusammensetzung bzw. Strukturformel beschränkt. Bevorzugt sind hier jedoch Smectite, insbesondere Bentonite. Geeignete Schichtsilikate, die zur Gruppe der mit Wasser quellfähigen Smectite zählen, sind z.B. solche der allgemeinen Formeln

$$(OH)_4Si_{8-y}Al_y(Mg_xAl_{4-x})O_{20} \qquad \text{Montmorrilonit}$$

$$(OH)_4Si_{8-y}Al_y(Mg_{6-z}Li_z)O_{20} \qquad \text{Hectorit}$$

$$(OH)_4Si_{8-y}Al_y(Mg_{6-z}\,Al_z)O_{20} \qquad \text{Saponit}$$

mit x = 0 bis 4, y = 0 bis 2, z = 0 bis 6. Zusätzlich kann in das Kristallgitter der Schichtsilikate gemäß den vorstehenden Formeln geringe Mengen an Eisen eingebaut sein. Ferner können die Schichtsilikate aufgrund ihrer ionenaustauschenden Eigenschaften Wasserstoff-, Alkali-, Erdalkaliionen, insbesondere $Na^+$ und $Ca^{2+}$ enthalten. Die Hydratwassermenge liegt meist im Bereich von 8 bis 20 Gew.-% und ist vom Quellzustand bzw. von der Art der Bearbeitung abhängig. Brauchbare Schichtsilikate sind beispielsweise aus **US 3,966,629, US 4,062,647, EP 0026529 A1** und **EP 0028432 A1** bekannt. Vorzugsweise werden Schichtsilikate verwendet, die aufgrund einer Alkalibehandlung weitgehend frei

von Caldumionen und stark färbenden Eisenionen sind.

Zu den bevorzugten Buildersubstanzen gehören auch amorphe Natriumsilikate mit einem Modul $Na_2O$ : $SiO_2$ von 1 : 2 bis 1 : 3,3, vorzugsweise von 1 : 2 bis 1 : 2,8 und insbesondere von 1 : 2 bis 1 : 2,6, welche löseverzögert sind und Sekundärwascheigenschaften aufweisen. Die Löseverzögerung gegenüber herkömmlichen amorphen Natriumsilikaten kann dabei auf verschiedene Weise, beispielsweise durch Oberflächenbehandlung, Compoundierung, Kompaktierung/Verdichtung oder durch Übertrocknung hervorgerufen worden sein. Im Rahmen dieser Erfindung wird unter dem Begriff "amorph" auch "röntgenamorph" verstanden. Dies heißt, daß die Silikate bei Röntgenbeugungsexperimenten keine scharfen Röntgenreflexe liefern, wie sie für kristalline Substanzen typisch sind, sondern allenfalls ein oder mehrere Maxima der gestreuten Röntgenstrahlung, die eine Breite von mehreren Gradeinheiten des Beugungswinkels aufweisen. Es kann jedoch sehr wohl sogar zu besonders guten Buildereigenschaften führen, wenn die Silikatpartikel bei Elektronenbeugungsexperimenten verwaschene oder sogar scharfe Beugungsmaxima liefern. Dies ist so zu interpretieren, daß die Produkte mikrokristalline Bereiche der Größe 10 bis einige Hundert nm aufweisen, wobei Werte bis max. 50 nm und insbesondere bis max. 20 nm bevorzugt sind. Derartige sogenannte röntgenamorphe Silikate, welche ebenfalls eine Löseverzögerung gegenüber den herkömmlichen Wassergläsern aufweisen, werden beispielsweise in der deutschen Patentanmeldung **DE 4400024 A1** beschrieben. Insbesondere bevorzugt sind verdichtete/kompaktierte amorphe Silikate, compoundierte amorphe Silikate und übertrocknete röntgenamorphe Silikate.

Selbstverständlich ist auch ein Einsatz der allgemein bekannten Phosphate als Buildersubstanzen möglich, sofern ein derartiger Einsatz nicht aus ökologischen Gründen vermieden werden sollte. Geeignet sind insbesondere die Natriumsalze der Orthophosphate, der Pyrophosphate und insbesondere der Tripolyphosphate. Ihr Gehalt beträgt im allgemeinen nicht mehr als 25 Gew.-%, vorzugsweise nicht mehr als 20 Gew.-%, jeweils bezogen auf das fertige Mittel. In einigen Fällen hat es sich gezeigt, daß insbesondere Tripolyphosphate schon in geringen Mengen bis maximal 10 Gew.-%, bezogen auf das fertige Mittel, in Kombination mit anderen Buildersubstanzen zu einer synergistischen Verbesserung des Sekundärwaschvermögens führen.

<u>Co-Builder</u>

Brauchbare organische Gerüstsubstanzen, die als Co-Builder in Frage kommen, sind beispielsweise die in Form ihrer Natriumsalze einsetzbaren Polycarbonsäuren, wie Citronensäure, Adipinsäure, Bernsteinsäure, Glutarsäure, Weinsäure, Zuckersäuren, Aminocarbonsäuren, Nitrilotriessigsäure (NTA), sofern ein derartiger Einsatz aus ökologischen Gründen nicht zu beanstanden ist, sowie Mischungen aus diesen. Bevorzugte Salze sind die Salze der Polycarbonsäuren wie Citronensäure, Adipinsäure, Bernsteinsäure, Glutarsäure, Weinsäure, Zuckersäuren und Mischungen aus diesen. Auch die Säuren an sich können eingesetzt werden. Die Säuren besitzen neben ihrer Builderwirkung typischerweise auch die Eigenschaft einer Säuerungskomponente und dienen somit auch zur Einstellung eines niedrigeren und milderen pH-Wertes von Wasch- oder Reinigungsmitteln. Insbesondere sind hierbei Citronensäure, Bemsteinsäure, Glutarsäure, Adipinsäure, Gluconsäure und beliebige Mischungen aus diesen zu nennen.

Weitere geeignete organische Buildersubstanzen sind Dextrine, beispielsweise Oligomere bzw. Polymere von Kohlenhydraten, die durch partielle Hydrolyse von Stärken erhalten werden können. Die Hydrolyse kann nach üblichen, beispielsweise säure- oder enzymkatalysierten Verfahren durchgeführt werden. Vorzugsweise handelt es sich um Hydrolyseprodukte mit mittleren Molmassen im Bereich von 400 bis 500 000. Dabei ist ein Polysaccharid mit einem Dextrose-Äquivalent (DE) im Bereich von 0,5 bis 40, insbesondere von 2 bis 30 bevorzugt, wobei DE ein gebräuchliches Maß für die reduzierende Wirkung eines Polysaccharids im Vergleich zu Dextrose, welche ein DE von 100 besitzt, ist. Brauchbar sind sowohl Maltodextrine mit einem DE zwischen 3 und 20 und Trockenglucosesirupe mit einem DE zwischen 20 und 37 als auch sogenannte Gelbdextrine und Weißdextrine mit höheren Molmassen im Bereich von 2 000 bis 30 000. Ein bevorzugtes Dextrin ist in der britischen Patentanmeldung GB **9419091 A1** beschrieben. Bei den oxidierten Derivaten derartiger Dextrine handelt es sich um deren Umsetzungsprodukte mit Oxidationsmitteln, welche in der Lage sind, mindestens eine Alkoholfunktion des Saccharidrings zur Carbonsäurefunktion zu oxidieren. Derartige oxidierte Dextrine und Verfahren ihrer Herstellung sind beispielsweise aus den europäischen Patentanmeldungen **EP 0232202 A1, EP 0427349 A1, EP 0472042 A1** und **EP 0542496 A1** sowie den internationalen Patentanmeldungen **WO 92/18542, WO 93/08251, WO 93/16110, WO 94/28030, WO 95/07303, WO 95/12619** und **WO 95/20608** bekannt. Ebenfalls geeignet ist ein oxidiertes Oligosaccharid gemäß der deutschen Patentanmeldung **DE 19600018 A1**. Ein an $C_6$ des Saccharidrings oxidiertes Produkt kann besonders vorteilhaft sein.

Weitere geeignete Cobuilder sind Oxydisuccinate und andere Derivate von Disuccinaten, vorzugsweise Ethylendiamindisuccinat. Besonders bevorzugt sind in diesem Zusammenhang auch Glycerindisuccinate und Glycerintrisuccinate, wie sie beispielsweise in den US-amerikanischen Patentschriften **US 4,524,009, US 4,639,325,** in der europäischen Patentanmeldung **EP 0150930 A1** und der japanischen Patentanmeldung **JP 93/339896** beschrieben werden. Geeignete Einsatzmengen liegen in zeolithhaltigen und/oder silikathaltigen Formulierungen bei 3 bis 15 Gew.-%. Weitere brauchbare organische Cobuilder sind beispielsweise acetylierte Hydroxycarbonsäuren bzw. deren Salze, welche gegebenenfalls auch in Lactonform vorliegen können und welche mindestens 4 Kohlenstoffatome und mindestens eine Hydroxygruppe sowie maximal zwei Säuregruppen enthalten. Derartige Cobuilder werden beispielsweise in der internationalen Patentanmeldung **WO 95/20029** beschrieben.

Geeignete polymere Polycarboxylate sind beispielsweise die Natriumsalze der Polyacrylsäure oder der Polymethacrylsäure, beispielsweise solche mit einer relativen Molekülmasse von 800 bis 150 000 (auf Säure bezogen und jeweils gemessen gegen Polystyrolsulfonsäure). Geeignete copolymere Polycarboxylate sind insbesondere solche der Acrylsäure mit Methacrylsäure und der Acrylsäure oder Methacrylsäure mit Maleinsäure. Als besonders geeignet haben sich Copolymere der Acrylsäure mit Maleinsäure erwiesen, die 50 bis 90 Gew.-% Acrylsäure und 50 bis 10 Gew.-% Maleinsäure enthalten. Ihre relative Molekülmasse, bezogen auf freie Säuren, beträgt im allgemeinen 5 000 bis 200 000, vorzugsweise 10 000 bis 120 000 und insbesondere 50 000 bis 100 000 (jeweils gemessen gegen Polystyrolsulfonsäure). Die (co-)polymeren Polycarboxylate können entweder als Pulver oder als wässrige Lösung eingesetzt werden, wobei 20 bis 55 Gew.-%ige wässrige Lösungen bevorzugt sind. Granulare Polymere werden zumeist nachträglich zu einem oder mehreren Basisgranulaten zugemischt. Insbesondere bevorzugt sind auch biologisch abbaubare Polymere aus mehr als zwei verschiedenen Monomereinheiten, beispielsweise solche, die gemäß der **DE 4300772 A1** als Monomere Salze der Acrylsäure und der Maleinsäure sowie Vinylalkohol bzw. Vinylalkohol-Derivate oder gemäß der **DE 4221381 C2** als Monomere Salze der Acrylsäure und der 2-Alkylallylsulfonsäure sowie Zucker-Derivate enthalten. Weitere bevorzugte Copolymere sind solche, die in den deutschen Patentanmeldungen **DE 4303320 A1** und **DE 4417734 A1** beschrieben werden und als Monomere vorzugsweise Acrolein und Acrylsäure/Acrylsäuresalze bzw. Acrolein und Vinylacetat aufweisen. Ebenso sind als weitere bevorzugte Buildersubstanzen polymere Aminodicarbonsäuren, deren Salze oder deren Vorläufersubstanzen zu nennen. Besonders bevorzugt sind Polyasparaginsäuren bzw. deren Salze und Derivate.

Weitere geeignete Buildersubstanzen sind Polyacetale, welche durch Umsetzung von Dialdehyden mit Polyolcarbonsäuren, welche 5 bis 7 C-Atome und mindestens 3 Hydroxylgruppen aufweisen, beispielsweise wie in der europäischen Patentanmeldung **EP 0280223 A1** beschrieben, erhalten werden können. Bevorzugte Polyacetale werden aus Dialdehyden wie Glyoxal, Glutaraldehyd, Terephthalaldehyd sowie deren Gemischen und aus Polyolcarbonsäuren wie Gluconsäure und/oder Glucoheptonsäure erhalten.

Öl- und fettlösende Stoffe

Zusätzlich können die Mittel auch Komponenten enthalten, welche die Öl- und Fett-Auswaschbarkeit aus Textilien positiv beeinflussen. Zu den bevorzugten öl- und fettlösenden Komponenten zählen beispielsweise nichtionische Celluloseether wie Methylcellulose und Methylhydroxypropylcellulose mit einem Anteil an Methoxyl-Gruppen von 15 bis 30 Gew.-% und an Hydroxypropoxyl-Gruppen von 1 bis 15 Gew.-%, jeweils bezogen auf den nichtionischen Celluloseether, sowie die aus dem Stand der Technik bekannten Polymere der Phthalsäure und/oder der Terephthalsäure bzw. von deren Derivaten, insbesondere Polymere aus Ethylenterephthalaten und/oder Polyethylenglykolterephthalaten oder anionisch und/oder nichtionisch modifizierten Derivaten von diesen. Besonders bevorzugt von diesen sind die sulfonierten Derivate der Phthalsäure- und der Terephthalsäure-Polymere.

Bleichmittel und Bleichaktivatoren

Unter den als Bleichmittel dienenden, in Wasser $H_2O_2$ liefernden Verbindungen haben das Natriumperborattetrahydrat und das Natriumperboratmonohydrat besondere Bedeutung. Weitere brauchbare Bleichmittel sind beispielsweise Natriumpercarbonat, Peroxypyrophosphate, Citratperhydrate sowie $H_2O_2$ liefernde persaure Salze oder Persäuren, wie Perbenzoate, Peroxophthalate, Diperazelainsäure, Phthaloiminopersäure oder Diperdodecandisäure. Der Gehalt der Mittel an Bleichmitteln beträgt vorzugsweise 5 bis 35 Gew.-% und insbesondere bis 30 Gew.-%, wobei vorteilhafterweise Perboratmonohydrat oder Percarbonat eingesetzt wird.

Als Bleichaktivatoren können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. iso-NOBS), Carbonsäureanhydride, insbesondere Phthalsäureanhydrid, acylierte mehrwertige Alkohole, insbesondere Triacetin, Ethylenglykoldiacetat, 2,5-Diacetoxy-2,5-dihydrofuran und die aus den deutschen Patentanmeldungen **DE 19616693 A1** und **DE 19616767 A1** bekannten Enolester sowie acetyliertes Sorbitol und Mannitol beziehungsweise deren in der europäischen Patentanmeldung **EP 0525239 A1** beschriebene Mischungen (SORMAN), acylierte Zuckerderivate, insbesondere Pentaacetylglukose (PAG), Pentaacetylfruktose, Tetraacetylxylose und Octaacetyllactose sowie acetyliertes, gegebenenfalls N-alkyliertes Glucamin und Gluconolacton, und/oder N-acylierte Lactame, beispielsweise N-Benzoylcaprolactam, die aus den internationalen Patentanmeldungen **WO 94/27970, WO 94/28102, WO 94/28103, WO 95/00626, WO 95/14759** und **WO 95/17498** bekannt sind. Die aus der deutschen Patentanmeldung **DE 19616769 A1** bekannten hydrophil substituierten Acylacetale und die in der deutschen Patentanmeldung **DE 196 16 770** sowie der internationalen Patentanmeldung **WO 95/14075** beschriebenen Acyllactame werden ebenfalls bevorzugt eingesetzt. Auch die aus der deutschen Patentanmeldung **DE 4443177 A1** bekannten Kombinationen konventioneller Bleichaktivatoren können eingesetzt werden. Derartige Bleichaktivatoren sind im üblichen Mengenbereich, vorzugsweise in Mengen von 1 Gew.-% bis 10 Gew.-%, insbesondere 2 Gew.-% bis 8 Gew.-%, bezogen auf gesamtes Mittel, enthalten. Zusätzlich zu den oben aufgeführten konventionellen Bleichaktivatoren oder an deren Stelle können auch die aus den europäischen Patentschriften **EP 0446982 B1** und **EP 0453 003 B1** bekannten Sulfonimine und/oder bleichverstärkende Übergangsmetallsalze beziehungsweise Übergangsmetallkomplexe als sogenannte Bleichkatalysatoren enthalten sein. Zu den in Frage kommenden Übergangsmetallverbindungen gehören insbesondere die aus der deutschen Patentanmeldung **DE 19529905 A1** bekannten Mangan-, Eisen-, Kobalt-, Ruthenium- oder Molybdän-Salenkomplexe und deren aus der deutschen Patentanmeldung **DE 19620267 A1** bekannte N-Analogverbindungen, die aus der deutschen Patentanmeldung **DE 19536082 A1** bekannten Mangan-, Eisen-, Kobalt-, Ruthenium- oder Molybdän-Carbonylkomplexe, die in der deutschen Patentanmeldung **DE 19605688 A1** beschriebenen Mangan-, Eisen-, Kobalt-, Ruthenium-, Molybdän-, Titan-, Vanadium- und Kupfer-Komplexe mit stickstoffhaltigen Tripod-Uganden, die aus der deutschen Patentanmeldung **DE 19620411 A1** bekannten Kobalt-, Eisen-, Kupfer- und Ruthenium-Aminkomplexe, die in der deutschen Patentanmeldung **DE 4416438 A1** beschriebenen Mangan-, Kupfer- und Kobalt-Komplexe, die in der europäischen Patentanmeldung **EP 0272030 A1** beschriebenen Kobalt-Komplexe, die aus der europäischen Patentanmeldung **EP 0693550 A1** bekannten Mangan-Komplexe, die aus der europäischen Patentschrift **EP 0392592 A1** bekannten Mangan-, Eisen-, Kobalt- und Kupfer-Komplexe und/oder die in der europäischen Patentschrift **EP 0443651 B1** oder den europäischen Patentanmeldungen **EP 0458397 A1, EP 0458398 A1, EP 0549271 A1, EP 0549272 A1, EP 0544490 A1** und **EP 0544519 A1** beschriebenen Mangan-Komplexe. Kombinationen aus Bleichaktivatoren und Übergangsmetall-Bleichkatalysatoren sind beispielsweise aus der deutschen Patentanmeldung **DE 19613103 A1** und der intemationalen Patentanmeldung **WO 95/27775** bekannt. Bleichverstärkende Übergangsmetallkomplexe, insbesondere mit den Zentralatomen Mn, Fe, Co, Cu, Mo, V, Ti und/oder Ru, werden in üblichen Mengen, vorzugsweise in einer Menge bis zu 1 Gew.-%, insbesondere von 0,0025 Gew.-% bis 0,25 Gew.-% und besonders bevorzugt von 0,01 Gew.-% bis 0,1 Gew.-%, jeweils bezogen auf gesamtes Mittel, eingesetzt.

Enzyme und Enzymstabilisatoren

Als Enzyme kommen insbesondere solche aus der Klasse der Hydrolasen, wie der Proteasen, Esterasen, Lipasen bzw. lipolytisch wirkenden Enzyme, Amylasen, Cellulasen bzw. andere Glykosylhydrolasen und Gemische der genannten Enzyme in Frage. Alle diese Hydrolasen tragen in der Wäsche zur Entfernung von Verfleckungen, wie protein-, fett- oder stärkehaltigen Verfleckungen, und Vergrauungen bei. Cellulasen und andere Glykosylhydrolasen können durch das Entfernen von Pilling und Mikrofibrillen zur Farberhaltung und zur Erhöhung der Weichheit des Textils beitragen. Zur Bleiche bzw. zur Hemmung der Farbübertragung können auch Oxidoreduktasen eingesetzt werden. Besonders gut geeignet sind aus Bakterienstämmen oder Pilzen, wie Bacillus subtilis, Bacillus licheniformis, Streptomyces griseus und Humicola insolens gewonnene enzymatische Wirkstoffe. Vorzugsweise werden Proteasen vom Subtilisin-Typ und insbesondere Proteasen, die aus Bacillus lentus gewonnen werden, eingesetzt. Dabei sind Enzymmischungen, beispielsweise aus Protease und Amylase oder Protease und Lipase bzw. lipolytisch wirkenden Enzymen oder Protease und Cellulase oder aus Cellulase und Lipase bzw. lipolytisch wirkenden Enzymen oder aus Protease, Amylase und Lipase bzw. lipolytisch wirkenden Enzymen oder Protease, Lipase bzw. lipolytisch wirkenden Enzymen und Cellulase, insbesondere jedoch Protease- und/oder Lipase-haltige Mischungen bzw. Mischungen mit lipolytisch wirkenden Enzymen von besonderem Interesse. Beispiele für derartige lipolytisch wirkende Enzyme sind die bekannten Cutinasen. Auch Peroxidasen oder Oxidasen haben sich in einigen Fällen als geeignet erwiesen. Zu den geeigneten Amylasen zählen insbesondere $\alpha$-Amylasen, Iso-Amylasen, Pullulanasen und Pektinasen. Als Cellulasen werden vorzugsweise Cellobiohydrolasen, Endoglucanasen und $\beta$-Glucosidasen, die auch Cellobiasen genannt werden, bzw. Mischungen aus diesen eingesetzt. Da sich die verschiedenen Cellulase-Typen durch ihre CMCase- und Avicelase-Aktivitäten unterscheiden, können durch gezielte Mischungen der Cellulasen die gewünschten Aktivitäten eingestellt werden.

Die Enzyme können an Trägerstoffen adsorbiert und/oder in Hüllsubstanzen eingebettet sein, um sie gegen vorzeitige Zersetzung zu schützen. Der Anteil der Enzyme, Enzymmischungen oder Enzymgranulate kann beispielsweise etwa 0,1 bis 5 Gew.-%, vorzugsweise 0,1 bis etwa 2 Gew.-% betragen.

Zusätzlich zu den mono- und polyfunktionellen Alkoholen können die Mittel weitere Enzymstabilisatoren enthalten. Beispielsweise können 0,5 bis 1 Gew.-% Natriumformiat eingesetzt werden. Möglich ist auch der Einsatz von Proteasen, die mit löslichen Calciumsalzen und einem Calciumgehalt von vorzugsweise etwa 1,2 Gew.-%, bezogen auf das Enzym, stabilisiert sind. Außer Calciumsalzen dienen auch Magnesiumsalze als Stabilisatoren. Besonders

vorteilhaft ist jedoch der Einsatz von Borverbindungen, beispielsweise von Borsäure, Boroxid, Borax und anderen Alkalimetallboraten wie den Salzen der Orthoborsäure ($H_3BO_3$), der Metaborsäure ($HBO_2$) und der Pyroborsäure (Tetraborsäure $H_2B_4O_7$).

Vergrauungsinhibitoren

Vergrauungsinhibitoren haben die Aufgabe, den von der Faser abgelösten Schmutz in der Flotte suspendiert zu halten und so das Wiederaufziehen des Schmutzes zu verhindern. Hierzu sind wasserlösliche Kolloide meist organischer Natur geeignet, beispielsweise die wasserlöslichen Salze polymerer Carbonsäuren, Leim, Gelatine, Salze von Ethercarbonsäuren oder Ethersulfonsäuren der Stärke oder der Cellulose oder Salze von sauren Schwefelsäureestern der Cellulose oder der Stärke. Auch wasserlösliche, saure Gruppen enthaltende Polyamide sind für diesen Zweck geeignet. Weiterhin lassen sich lösliche Stärkepräparate und andere als die obengenannten Stärkeprodukte verwenden, z.B. abgebaute Stärke, Aldehydstärken usw.. Auch Polyvinylpyrrolidon ist brauchbar. Bevorzugt werden jedoch Celluloseether, wie Carboxymethylcellulose (Na-Salz), Methylcellulose, Hydroxyalkylcellulose und Mischether, wie Methylhydroxyethylcellulose, Methylhydroxypropylcellulose, Methylcarboxymethylcellulose und deren Gemische, sowie Polyvinylpyrrolidon beispielsweise in Mengen von 0,1 bis 5 Gew.-%, bezogen auf die Mittel, eingesetzt.

Optische Aufheller

Die Mittel können als optische Aufheller Derivate der Diaminostilbendisulfonsäure bzw. deren Alkalimetallsalze enthalten. Geeignet sind z.B. Salze der 4,4'-Bis(2-anilino-4-morpholino-1,3,5-triazinyl-6-amino)stilben-2,2'-disulfonsäure oder gleichartig aufgebaute Verbindungen, die anstelle der Morpholino-Gruppe eine Diethanolaminogruppe, eine Methylaminogruppe, eine Anilinogruppe oder eine 2-Methoxyethylaminogruppe tragen. Weiterhin können Aufheller vom Typ der substituierten Diphenylstyryle anwesend sein, z.B. die Alkalisalze des 4,4'-Bis(2-sulfostyryl)-diphenyls, 4,4'-Bis(4-chlor-3-sulfostyryl)-diphenyls, oder 4-(4-Chlorstyryl)-4'-(2-sulfostyryl)-diphenyls. Auch Gemische der vorgenannten Aufheller können verwendet werden. Einheitlich weiße Granulate werden erhalten, wenn die Mittel außer den üblichen Aufhellern in üblichen Mengen, beispielsweise zwischen 0,1 und 0,5 Gew.-%, vorzugsweise zwischen 0,1 und 0,3 Gew.-%, auch geringe Mengen, beispielsweise $10^{-6}$ bis $10^{-3}$ Gew.-%, vorzugsweise um $10^{-5}$ Gew.-%, eines blauen Farbstoffs enthalten. Ein besonders bevorzugter Farbstoff ist Tinolux® (Handelsprodukt der Ciba-Geigy).

Polymere

Als schmutzabweisende Polymere ("soil repellants") kommen solche Stoffe in Frage, die vorzugsweise Ethylenterephthalat- und/oder Polyethylenglycolterephthalatgruppen enthalten, wobei das Molverhältnis Ethylenterephthalat zu Polyethylenglycolterephthalat im Bereich von 50 : 50 bis 90 : 10 liegen kann. Das Molekulargewicht der verknüpfenden Polyethylenglycoleinheiten liegt insbesondere im Bereich von 750 bis 5000, d.h., der Ethoxylierungsgrad der Polyethylenglycolgruppenhaltigen Polymere kann ca. 15 bis 100 betragen. Die Polymeren zeichnen sich durch ein durchschnittliches Molekulargewicht von etwa 5000 bis 200.000 aus und können eine Block-, vorzugsweise aber eine Random-Struktur aufweisen. Bevorzugte Polymere sind solche mit Molverhältnissen Ethylenterephthalat/Polyethylenglycolterephthalat von etwa 65 : 35 bis etwa 90 : 10, vorzugsweise von etwa 70 : 30 bis 80 : 20. Weiterhin bevorzugt sind solche Polymeren, die verknüpfende Polyethylenglycoleinheiten mit einem Molekulargewicht von 750 bis 5000, vorzugsweise von 1000 bis etwa 3000 und ein Molekulargewicht des Polymeren von etwa 10.000 bis etwa 50.000 aufweisen. Beispiele für handelsübliche Polymere sind die Produkte Milease® T (ICI) oder Repelotex® SRP 3 (Rhône-Poulenc).

Entschäumer

Als Entschäumer können wachsartige Verbindungen eingesetzt werden. Als "wachsartig" werden solche Verbindungen verstanden, die einen Schmelzpunkt bei Atmosphärendruck über 25 °C (Raumtemperatur), vorzugsweise über 50 °C und insbesondere über 70 °C aufweisen. Die wachsartigen Entschäumersubstanzen sind in Wasser praktisch nicht löslich, d.h. bei 20 °C weisen sie in 100 g Wasser eine Löslichkeit unter 0,1 Gew.-% auf. Prinzipiell können alle aus dem Stand der Technik bekannten wachsartigen Entschäumersubstanzen enthalten sein. Geeignete wachsartige Verbindungen sind beispielsweise Bisamide, Fettalkohole, Fettsäuren, Carbonsäureester von ein- und mehrwertigen Alkoholen sowie Paraffinwachse oder Mischungen derselben. Alternativ können natürlich auch die für diesen Zweck bekannten Silikonverbindungen eingesetzt werden.

Geeignete Paraffinwachse stellen im allgemeinen ein komplexes Stoffgemisch ohne scharfen Schmelzpunkt dar. Zur Charakterisierung bestimmt man üblicherweise seinen Schmelzbereich durch Differential-Thermo-Analyse (DTA), wie in **"The Analyst" 87 (1962), 420,** beschrieben, und/oder seinen Erstarrungspunkt. Darunter versteht man

die Temperatur, bei der das Paraffin durch langsames Abkühlen aus dem flüssigen in den festen Zustand übergeht. Dabei sind bei Raumtemperatur vollständig flüssige Paraffine, das heißt solche mit einem Erstarrungspunkt unter 25 °C, erfindungsgemäß nicht brauchbar. Zu den Weichwachsen, die einen Schmelzpunkt im Bereich von 35 bis 50 °C aufweisen, zählen vorzugsweise der Gruppe der Petrolate und deren Hydrierprodukte. Sie setzen sich aus mikrokristallinen Paraffinen und bis zu 70 Gew.-% Öl zusammen, besitzen eine salbenartige bis plastisch feste Konsistenz und stellen bitumenfreie Rückstände aus der Erdölverarbeitung dar. Besonders bevorzugt sind Destillationsrückstände (Petrolatumstock) bestimmter paraffinbasischer und gemischtbasischer Rohöle, die zu Vaseline weiterverarbeitet werden. Vorzugsweise handelt es sich weiterhin um aus Destillationsrückständen paraffin- und gemischtbasyischer Rohöle und Zylinderöldestillate mittels Lösungsmittel abgeschiedene bitumenfreie, ölartige bis feste Kohlenwasserstoffe. Sie sind von halbfester, zügiger, klebriger bis plastisch-fester Konsistenz und besitzen Schmelzpunkte zwischen 50 und 70 °C. Diese Petrolate stellen die wichtigste Ausgangsbasis für die Herstellung von Mikrowachsen dar. Weiterhin geeignet sind die aus hochviskosen, paraffinhaltigen Schmieröldestillaten bei der Entparaffinierung abgeschiedenen festen Kohlenwasserstoffen mit Schmelzpunkten zwischen 63 und 79 °C. Bei diesen Petrolaten handelt es sich um Gemische aus mikrokristallinen Wachsen und hochschmelzenden n-Paraffinen. Eingesetzt werden können beispielsweise die aus **EP 0309931 A1** bekannten Paraffinwachsgemische aus beispielsweise 26 Gew.-% bis 49 Gew.-% mikrokristallinem Paraffinwachs mit einem Erstarrungspunkt von 62 °C bis 90 °C, 20 Gew.-% bis 49 Gew.-% Hartparaffin mit einem Erstarrungspunkt von 42 °C bis 56 °C und 2 Gew.-% bis 25 Gew.-% Weichparaffin mit einem Erstarrungspunkt von 35 °C bis 40 °C. Vorzugsweise werden Paraffine bzw. Paraffingemische verwendet, die im Bereich von 30 °C bis 90 °C erstarren. Dabei ist zu beachten, daß auch bei Raumtemperatur fest erscheinende Paraffinwachsgemische unterschiedliche Anteile an flüssigem Paraffin enthalten können. Bei den erfindungsgemäß brauchbaren Paraffinwachsen liegt dieser Flüssiganteil so niedrig wie möglich und fehlt vorzugsweise ganz. So weisen besonders bevorzugte Paraffinwachsgemische bei 30 °C einen Flüssiganteil von unter 10 Gew.-%, insbesondere von 2 Gew.-% bis 5 Gew.-%, bei 40 °C einen Flüssiganteil von unter 30 Gew.-%, vorzugsweise von 5 Gew.-% bis 25 Gew.-% und insbesondere von 5 Gew.-% bis 15 Gew.-%, bei 60 °C einen Flüssiganteil von 30 Gew.-% bis 60 Gew.-%, insbesondere von 40 Gew.-% bis 55 Gew.-%, bei 80 °C einen Flüssiganteil von 80 Gew.-% bis 100 Gew.-%, und bei 90 °C einen Flüssiganteil von 100 Gew.-% auf. Die Temperatur, bei der ein Flüssiganteil von 100 Gew.-% des Paraffinwachses erreicht wird, liegt bei besonders bevorzugten Paraffinwachsgemischen noch unter 85 °C, insbesondere bei 75 °C bis 82 °C. Bei den Paraffinwachsen kann es sich um Petrolatum, mikrokristalline Wachse bzw. hydrierte oder partiell hydrierte Paraffinwachse handeln.

Geeignete Bisamide als Entschäumer sind solche, die sich von gesättigten Fettsäuren mit 12 bis 22, vorzugsweise 14 bis 18 C-Atomen sowie von Alkylendiaminen mit 2 bis 7 C-Atomen ableiten. Geeignete Fettsäuren sind Laurin-, Myristin-, Stearin-, Arachin- und Behensäure sowie deren Gemische, wie sie aus natürlichen Fetten beziehungsweise gehärteten Ölen, wie Talg oder hydriertem Palmöl, erhältlich sind. Geeignete Diamine sind beispielsweise Ethylendiamin, 1,3-Propylendiamin, Tetramethylendiamin, Pentamethylendiamin, Hexamethylendiamin, p-Phenylendiamin und Toluylendiamin. Bevorzugte Diamine sind Ethylendiamin und Hexamethylendiamin. Besonders bevorzugte Bisamide sind Bismyristoylethylendiamin, Bispalmitoylethylendiamin, Bisstearoylethylendiamin und deren Gemische sowie die entsprechenden Derivate des Hexamethylendiamins.

Geeignete Carbonsäureester als Entschäumer leiten sich von Carbonsäuren mit 12 bis 28 Kohlenstoffatomen ab. Insbesondere handelt es sich um Ester von Behensäure, Stearinsäure, Hydroxystearinsäure, Ölsäure, Palmitinsäure, Myristinsäure und/oder Laurinsäure. Der Alkoholteil des Carbonsäureesters enthält einen ein- oder mehrwertigen Alkohol mit 1 bis 28 Kohlenstoffatomen in der Kohlenwasserstoffkette. Beispiele von geeigneten Alkoholen sind Behenylalkohol, Arachidylalkohol, Kokosalkohol, 12-Hydroxystearylalkohol, Oleylalkohol und Laurylalkohol sowie Ethylenglykol, Glycerin, Polyvinylalkohol, Saccharose, Erythrit, Pentaerythrit, Sorbitan und/oder Sorbit. Bevorzugte Ester sind solche von Ethylenglykol, Glycerin und Sorbitan, wobei der Säureteil des Esters insbesondere aus Behensäure, Stearinsäure, Ölsäure, Palmitinsäure oder Myristinsäure ausgewählt wird. In Frage kommende Ester mehrwertiger Alkohole sind beispielsweise Xylitmonopalmitat, Pentarythritmonostearat, Glycerin-monostearat, Ethylenglykolmonostearat und Sorbitanmonostearat, Sorbitanpalmitat, Sorbitanmonolaurat, Sorbitandilaurat, Sorbitandistearat, Sorbitandibehenat, Sorbitandioleat sowie gemischte Talgalkylsorbitanmono- und -diester. Brauchbare Glycerinester sind die Mono-, Di- oder Triester von Glycerin und genannten Carbonsäuren, wobei die Mono- oder Dieester bevorzugt sind. Glycerinmonostearat, Glycerinmonooleat, Glycerinmonopalmitat, Glycerinmonobehenat und Glycerindistearat sind Beispiele hierfür. Beispiele für geeignete natürliche Ester als Entschäumer sind Bienenwachs, das hauptsächlich aus den Estern $CH_3(CH_2)_{24}COO(CH_2)_{27}CH_3$ und $CH_3(CH_2)_{26}COO(CH_2)_{25}CH_3$ besteht, und Carnaubawachs, das ein Gemisch von Carnaubasäurealkylestern, oft in Kombination mit geringen Anteilen freier Carnaubasäure, weiteren langkettigen Säuren, hochmolekularen Alkoholen und Kohlenwasserstoffen, ist.

Geeignete Carbonsäuren als weitere Entschäumerverbindung sind insbesondere Behensäure, Stearinsäure, Ölsäure, Palmitinsäure, Myristinsäure und Laurinsäure sowie deren Gemische, wie sie aus natürlichen Fetten bzw. gegebenenfalls gehärteten Ölen, wie Talg oder hydriertem Palmöl, erhältlich sind. Bevorzugt sind gesättigte Fettsäuren mit 12 bis 22, insbesondere 18 bis 22 C-Atomen. In gleicher Weise können die entsprechenden Fettalkohole gleicher

C-Kettenlänge eingesetzt werden.

Weiterhin können zusätzlich Dialkylether als Entschäumer enthalten sein. Die Ether können asymmetrisch oder aber symmetrisch aufgebaut sein, d.h. zwei gleiche oder verschiedene Alkylketten, vorzugsweise mit 8 bis 18 Kohlenstoffatomen enthalten. Typische Beispiele sind Di-n-octylether, Di-i-octylether und Di-n-stearylether, insbesondere geeignet sind Diatky lether, die einen Schmelzpunkt über 25 °C, insbesondere über 40 °C aufweisen.

Weitere geeignete Entschäumerverbindungen sind Fettketone, die nach den einschlägigen Methoden der präparativen organischen Chemie erhalten werden können. Zu ihrer Herstellung geht man beispielsweise von Carbonsäuremagnesiumsalzen aus, die bei Temperaturen oberhalb von 300 °C unter Abspaltung von Kohlendioxid und Wasser pyrolysiert werden, beispielsweise gemäß der deutschen Offenlegungsschrift **DE 2553900 OS.** Geeignete Fettketone sind solche, die durch Pyrolyse der Magnesiumsalze von Laurinsäure, Myristinsäure, Palmitinsäure, Palmitoleinsäure, Stearinsäure, Ölsäure, Elaidinsäure, Petroselinsäure, Arachinsäure, Gadoleinsäure, Behensäure oder Erucasäure hergestellt werden.

Weitere geeignete Entschäumer sind Fettsäurepolyethylenglykolester, die vorzugsweise durch basisch homogen katalysierte Anlagerung von Ethylenoxid an Fettsäuren erhalten werden. Insbesondere erfolgt die Anlagerung von Ethylenoxid an die Fettsäuren in Gegenwart von Alkanolaminen als Katalysatoren. Der Einsatz von Alkanolaminen, speziell Triethanolamin, führt zu einer äußerst selektiven Ethoxylierung der Fettsäuren, insbesondere dann, wenn es darum geht, niedrig ethoxylierte Verbindungen herzustellen. Innerhalb der Gruppe der Fettsäurepolyethylenglykolester werden solche bevorzugt, die einen Schmelzpunkt über 25 °C, insbesondere über 40 °C aufweisen.

Innerhalb der Gruppe der wachsartigen Entschäumer werden besonders bevorzugt die beschriebenen Paraffinwachse alleine als wachsartige Entschäumer eingesetzt oder in Mischung mit einem der anderen wachsartigen Entschäumer, wobei der Anteil der Paraffinwachse in der Mischung vorzugsweise über 50 Gew.-% - bezogen auf wachsartige Entschäumermischung - ausmacht. Die Paraffinwachse können bei Bedarf auf Träger aufgebracht sein. Als Trägermaterial sind alle bekannten anorganischen und/oder organischen Trägermaterialien geeignet. Beispiele für typische anorganische Trägermaterialien sind Alkalicarbonate, Alumosilikate, wasserlösliche Schichtsilikate, Alkalisilikate, Alkalisulfate, beispielsweise Natriumsulfat, und Alkaliphosphate. Bei den Alkalisilikaten handelt es sich vorzugsweise um eine Verbindung mit einem Molverhältnis Alkalioxid zu $SiO_2$ von 1 : 1,5 bis 1 : 3,5. Die Verwendung derartiger Silikate resultiert in besonders guten Korneigenschaften, insbesondere hoher Abriebsstabilität und dennoch hoher Auflösungsgeschwindigkeit in Wasser. Zu den als Trägermaterial bezeichneten Alumosilikaten gehören insbesondere die Zeolithe, beispielsweise Zeolith NaA und NaX. Zu den als wasserlöslichen Schichtsilikaten bezeichneten Verbindungen gehören beispielsweise amorphes oder kristallines Wasserglas. Weiterhin können Silikate Verwendung finden, welche unter der Bezeichnung Aerosil® oder Sipernat® im Handel sind. Als organische Trägermaterialien kommen zum Beispiel filmbildende Polymere, beispielsweise Polyvinylalkohole, Polyvinylpyrrolidone, Poly(meth)acrylate, Polycarboxylate, Cellulosederivate und Stärke in Frage. Brauchbare Celluloseether sind insbesondere Alkalicarboxymethylcellulose, Methylcellulose, Ethylcellulose, Hydroxyethylcellulose und sogenannte Cellulosemischether, wie zum Beispiel Methylhydroxyethylcellulose und Methylhydroxypropylcellulose, sowie deren Mischungen. Besonders geeignete Mischungen sind aus Natrium-Carboxymethylcellulose und Methylcellulose zusammengesetzt, wobei die Carboxymethylcellulose üblicherweise einen Substitutionsgrad von 0,5 bis 0,8 Carboxymethylgruppen pro Anhydroglukoseeinheit und die Methylcellulose einen Substitutionsgrad von 1,2 bis 2 Methylgruppen pro Anhydroglukoseeinheit aufweist. Die Gemische enthalten vorzugsweise Alkalicarboxymethylcellulose und nichtionischen Celluloseether in Gewichtsverhältnissen von 80 : 20 bis 40 : 60, insbesondere von 75 : 25 bis 50 : 50. Als Träger ist auch native Stärke geeignet, die aus Amylose und Amylopectin aufgebaut ist. Als native Stärke wird Stärke bezeichnet, wie sie als Extrakt aus natürlichen Quellen zugänglich ist, beispielsweise aus Reis, Kartoffeln, Mais und Weizen. Native Stärke ist ein handelsübliches Produkt und damit leicht zugänglich. Als Trägermaterialien können einzeln oder mehrere der vorstehend genannten Verbindungen eingesetzt werden, insbesondere ausgewählt aus der Gruppe der Alkalicarbonate, Alkalisulfate, Alkaliphosphate, Zeolithe, wasserlösliche Schichtsilikate, Alkalisilikate, Polycarboxylate, Celluloseether, Polyacrylat/Polymethacrylat und Stärke. Besonders geeignet sind Mischungen von Alkalicarbonaten, insbesondere Natriumcarbonat, Alkalisilikaten, insbesondere Natriumsilikat, Alkalisulfaten, insbesondere Natriumsulfat und Zeolithen.

Geeignete Silicone sind übliche Organopolysiloxane, die einen Gehalt an feinteiliger Kieselsäure, die wiederum auch silaniert sein kann, aufweisen können. Derartige Organopolysiloxane sind beispielsweise in der Europäischen Patentanmeldung **EP 0496510 A1** beschrieben. Besonders bevorzugt sind Polydiorganosiloxane und insbesondere Polydimethylsiloxane, die aus dem Stand der Technik bekannt sind. Geeignete Polydiorganosiloxane weisen eine nahezu lineare Kette auf und weisen einen Oligomerisierungsgrad von 40 bis 1500 auf. Beispiele für geeignete Substituenten sind Methyl, Ethyl, Propyl, Isobutyl, tert. Butyl und Phenyl. Weiterhin geeignet sind amino-, fettsäure-, alkohol-, polyether-, epoxy-, fluor-, glykosidund/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silikaten handelt. In der Regel enthalten die Silicone im allgemeinen und die Polydiorganosiloxane im besonderen feinteilige Kieselsäure, die auch silaniert sein kann. Insbesondere geeignet sind im Sinne der vorliegenden Erfindung kieselsäurehaltige Dimethylpolysiloxane. Vorteilhafterweise haben die Polydiorganosiloxane eine Viskosität nach Brookfield bei 25 °C (Spindel 1, 10 Upm) im Bereich von 5000 mPas bis 30 000 mPas, insbesondere von 15 000 bis 25 000 mPas. Vorzugsweise werden die Silicone in Form ihrer wässrigen Emulsionen eingesetzt. In der Regel gibt man das Silicon zu vorgelegtem Wasser unter Rühren. Falls gewünscht kann man zur Erhöhung der Viskosität der wässrigen Siliconemulsionen Verdickungsmittel, wie sie aus dem Stand der Technik bekannt sind, zugeben. Diese können anorganischer und/oder organischer Natur sein, besonders bevorzugt werden nichtionische Celluloseether wie Methylcellulose, Ethylcellulose und Mischether wie Methylhydoxyethylcellulose, Methylhydroxypropylcellulose, Methylhydroxybutylcellulose sowie anionische Carboxycellulose-Typen wie das Carboxymethylcellulose-Natriumsalz (Abkürzung CMC). Insbsonders geeignete Verdicker sind Mischungen von CMC zu nichtionischen Celluloseethern im Gewichtsverhältnis 80 : 20 bis 40 : 60, insbesondere 75 : 25 bis 60 : 40. In der Regel und besonders bei Zugabe der beschriebenen Verdickermischungen empfehlen sich Einsatzkonzentrationen von cirka 0,5 bis 10, insbesondere von 2,0 bis 6 Gew.-% - berechnet als Verdickermischung und bezogen auf wässrige Siliconemulsion. Die Gehalt an Siliconen der beschriebenen Art in den wässrigen Emulsionen liegt vorteilhafterweise im Bereich von 5 bis 50 Gew.-%, insbesondere von 20 bis 40 Gew.-% - berechnet als Silicone und bezogen auf wässrige Siliconemulsion. Nach einer weiteren vorteilhaften Ausgestaltung erhalten die wässrigen Siliconlösungen als Verdicker Stärke, die aus natürlichen Quellen zugänglich ist, beispielsweise aus Reis, Kartoffeln, Mais und Weizen. Die Stärke ist vorteilhafterweise in Mengen von 0,1 bis zu 50 Gew.-% - bezogen auf Silicon-Emulsion - enthalten und insbesondere in Mischung mit den schon beschriebenen Verdickermischungen aus Natrium-Carboxymethylcellulose und einem nichtionischen Celluloseether in den schon genannten Mengen. Zur Herstellung der wässrigen Siliconemulsionen geht man zweckmäßigerweise so vor, daß man die gegebenenfalls vorhandenen Verdickungsmittel in Wasser vorquellen läßt, bevor die Zugabe der Silicone erfolgt. Das Einarbeiten der Silicone erfolgt zweckmäßigerweise mit Hilfe wirksamer Rühr- und Mischungsvorrichtungen.

Sprengmittel

Die festen Zubereitungen können des weiteren Spreng- oder Desintegrationsmittel enthalten. Hierunter sind Stoffe zu verstehen, die den Formkörpern zugegeben werden, um deren Zerfall beim Inkontaktbringen mit Wasser zu beschleunigen. Übersichten hierzu finden sich z.B. in **J.Pharm.Sci. 61 (1972), Römpp Chemilexikon, 9. Auflage, Band 6, S. 4440** sowie und **Voigt *"Lehrbuch der pharmazeutischen Technologie"* (6. Auflage, 1987, S. 182-184).** Diese Stoffe vergrößern bei Wasserzutritt ihr Volumen, wobei einerseits das Eigenvolumen vergrößert (Quellung), andererseits auch über die Freisetzung von Gasen ein Druck erzeugt werden kann, der die Tablette in kleinere Partikel zerfallen läßt. Altbekannte Desintegrationshilfsmittel sind beispielsweise Carbonat/Citronensäure-Systeme, wobei auch andere organische Säuren eingesetzt werden können. Quellende Desintegrationshilfsmittel sind beispielsweise synthetische Polymere wie gegebenenfalls quervernetztes Polyvinylpyrrolidon (PVP) oder natürliche Polymere bzw. modifizierte Naturstoffe wie Cellulose und Stärke und ihre Derivate, Alginate oder Casein-Derivate. Als bevorzugte Desintegrationsmittel werden im Rahmen der vorliegenden Erfindung Desintegrationsmittel auf Cellulosebasis eingesetzt. Reine Cellulose weist die formale Bruttozusammensetzung $(C_6H_{10}O_5)_n$ auf und stellt formal betrachtet ein β-1,4-Polyacetal von Cellobiose dar, die ihrerseits aus zwei Molekülen Glucose aufgebaut ist. Geeignete Cellulosen bestehen dabei aus ca. 500 bis 5000 Glucose-Einheiten und haben demzufolge durchschnittliche Molmassen von 50.000 bis 500.000. Als Desintegrationsmittel auf Cellulosebasis verwendbar sind im Rahmen der vorliegenden Erfindung auch Cellulose-Derivate, die durch polymeranaloge Reaktionen aus Cellulose erhältlich sind. Solche chemisch modifizierten Cellulosen umfassen dabei beispielsweise Produkte aus Veresterungen bzw. Veretherungen, in denen Hydroxy-Wasserstoffatome substituiert wurden. Aber auch Cellulosen, in denen die Hydroxylgruppen gegen funktionelle Gruppen, die nicht über ein Sauerstoffatom gebunden sind, ersetzt wurden, lassen sich als Cellutosederivate einsetzen. In die Gruppe der Cellulosederivate fallen beispielsweise Alkalicellulosen, Carboxymethylcellulose (CMC), Celluloseester und -ether sowie Aminocellulosen. Die genannten Cellulosederivate werden vorzugsweise nicht allein als Sprengmittel auf Cellulosebasis eingesetzt, sondern in Mischung mit Cellulose verwendet. Der Gehalt dieser Mischungen an Cellulosederivaten beträgt vorzugsweise unterhalb 50 Gew.-%, besonders bevorzugt unterhalb 20 Gew.-%, bezogen auf das Desintegrationsmittel auf Cellulosebasis. Besonders bevorzugt wird als Desintegrationsmittel auf Cellulosebasis reine Cellulose eingesetzt, die frei von Cellulosederivaten ist. Als weiteres Desintegrationsmittel auf Cellulosebasis oder als Bestandteil dieser Komponente kann mikrokristalline Cellulose verwendet werden. Diese mikrokristalline Cellulose wird durch partielle Hydrolyse von Cellulosen unter solchen Bedingungen erhalten, die nur die amorphen Bereiche (ca. 30% der Gesamt-Cellulosemasse) der Cellulosen angreifen und vollständig auflösen, die kristallinen Bereiche (ca. 70%) aber unbeschadet lassen. Eine nachfolgende Desaggregation der durch die Hydrolyse entstehenden mikrofeinen Cellulosen liefert die mikrokristallinen Cellulosen, die Primärteilchengrößen von ca. 5 µm aufweisen und beispielsweise zu Granulaten mit einer mittleren Teilchengröße von 200 µm kompaktierbar sind. Die Sprengmittel können im Formkörper makroskopisch betrachtet homogen verteilt vorliegen, mikroskopisch gesehen

bilden sie jedoch herstellungsbedingt Zonen erhöhter Konzentration. Sprengmittel, die im Sinne der Erfindung zugegen sein können, wie z.B. Kollidon, Alginsäure und deren Alkalisalze, amorphe oder auch teilweise kristalline Schichtsilikate (Bentonite), Polyacrylate, Polyethylenglycole sind beispielsweise den Druckschriften **WO 98/40462** (Rettenmaier), **WO 98/55583** und **WO 98/55590** (Unilever) und **WO 98/40463, DE 19709991** und **DE 19710254** (Henkel) zu entnehmen. Auf die Lehre dieser Schriften wird ausdrücklich Bezug genommen. Die Formkörper können die Sprengmittel in Mengen von 0,1 bis 25, vorzugsweise 1 bis 20 und insbesondere 5 bis 15 Gew.-% - bezogen auf die Formkörper enthalten.

Duftstoffe

Als Parfümöle bzw. Duftstoffe können einzelne Riechstoffverbindungen, z.B. die synthetischen Produkte vom Typ der Ester, Ether, Aldehyde, Ketone, Alkohole und Kohlenwasserstoffe verwendet werden. Riechstoffverbindungen vom Typ der Ester sind z.B. Benzylacetat, Phenoxyethylisobutyrat, p-tert.-Butylcyclohexylacetat, Unalylacetat, Dimethylbenzylcarbinylacetat, Phenylethylacetat, Linalylbenzoat, Benzylformiat, Ethylmethylphenylglycinat, Allylcydohexylpropionat, Styrallylpropionat und Benzylsalicylat. Zu den Ethern zählen beispielsweise Benzylethylether, zu den Aldehyden z.B. die linearen Alkanale mit 8-18 C-Atomen, Citral, Citronellal, Citronellyloxyacetaldehyd, Cydamenaldehyd, Hydroxycitronellal, Lilial und Bourgeonal, zu den Ketonen z.B. die Jonone, $\alpha$-Isomethylionon und Methylcedrylketon, zu den Alkoholen Anethol, Citronellol, Eugenol, Geraniol, Linalool, Phenylethylalkohol und Terpineol, zu den Kohlenwasserstoffen gehören hauptsächlich die Terpene wie Limonen und Pinen. Bevorzugt werden jedoch Mischungen verschiedener Riechstoffe verwendet, die gemeinsam eine ansprechende Duftnote erzeugen. Solche Parfümöle können auch natürliche Riechstoffgemische enthalten, wie sie aus pflanzlichen Quellen zugänglich sind, z.B. Pine-, Citrus-, Jasmin-, Patchouly-, Rosen- oder Ylang-Ylang-Öl. Ebenfalls geeignet sind Muskateller, Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl und Labdanumöl sowie Orangenblütenöl, Neroliol, Orangenschalenöl und Sandelholzöl.

Die Duftstoffe können direkt in die erfindungsgemäßen Mittel eingearbeitet werden, es kann aber auch vorteilhaft sein, die Duftstoffe auf Träger aufzubringen, welche die Haftung des Parfüms auf der Wäsche verstärken und durch eine langsamere Duftfreisetzung für langanhaltenden Duft der Textilien sorgen. Als solche Trägermaterialien haben sich beispielsweise Cyclodextrine bewährt, wobei die Cyclodextrin-Parfüm-Komplexe zusätzlich noch mit weiteren Hilfsstoffen beschichtet werden können.

Anorganische Salze

Weitere geeignete Inhaltsstoffe der Mittel sind wasserlösliche anorganische Salze wie Bicarbonate, Carbonate, amorphe Silikate, normale Wassergläser, welche keine herausragenden Buildereigenschaften aufweisen, oder Mischungen aus diesen; insbesondere werden Alkalicarbonat und/oder amorphes Alkalisilikat, vor allem Natriumsilikat mit einem molaren Verhältnis $Na_2O$ : $SiO_2$ von 1 : 1 bis 1 : 4,5, vorzugsweise von 1 : 2 bis 1 : 3,5, eingesetzt. Der Gehalt an Natriumcarbonat in den endzubereitungen beträgt dabei vorzugsweise bis zu 40 Gew.-%, vorteilhafterweise zwischen 2 und 35 Gew.-%. Der Gehalt der Mittel an Natriumsilikat (ohne besondere Buildereigenschaften) beträgt im allgemeinen bis zu 10 Gew.-% und vorzugsweise zwischen 1 und 8 Gew.-%. Als Füll- bzw. Stellmittel kann ferner beispielsweise Natriumsulfat in Mengen von 0 bis 10, insbesondere 1 bis 5 Gew.-% - bezogen auf Mittel - enthalten sein

**Haut- und Haarbehandlungsmittel**

Des weiteren kann es sich bei den oberflächenaktiven Zubereitungen auch um haut- oder Haarbehandlungsmittel handeln, die ebenfalls weitere für diese Mittel typische Hilfs- und Zusatzstoffe aufweisen können. Hierzu zählen beispielsweise milde Tenside, Ölkörper, Emulgatoren, Perlglanzwachse, Konsistenzgeber, Verdickungsmittel, Überfettungsmittel, Stabilisatoren, Polymere, Siliconverbindungen, Fette, Wachse, Lecithine, Phospholipide, biogene Wirkstoffe, UV-Lichtschutzfaktoren, Antioxidantien, Deodorantien, Antitranspirantien, Antischuppenmittel, Filmbildner, Quellmittel, Insektenrepellentien, Selbstbräuner, Tyrosininhibitoren (Depigmentierungsmittel), Hydrotrope, Solubilisatoren, Konservierungsmittel, Parfümöle, Farbstoffe und dergleichen.

Tenside

Als oberflächenaktive Stoffe können anionische, nichtionische, kationische und/oder amphotere bzw. amphotere Tenside enthalten sein, deren Anteil an den Mitteln üblicherweise bei etwa 1 bis 70, vorzugsweise 5 bis 50 und insbesondere 10 bis 30 Gew.-% beträgt. Typische Beispiele für anionische Tenside sind Seifen, Alkylbenzolsulfonate, Alkansulfonate, Olefinsulfonate, Alkylethersulfonate, Glycerinethersulfonate, $\alpha$-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl (ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Feealkoholpolyglycolemer, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk (en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Berlin, 1987, S. 54-124 oder J.Falbe (ed.), "Katalysatoren, Tenside und Mineralöladditive", Thieme Verlag, Stuttgart, 1978, S. 123-217** verwiesen. Typische Beispiele für besonders geeignete milde, d. h. besonders hautverträgliche Tenside sind Fettalkoholpolyglycolethersulfate, Monoglyceridsulfate, Monound/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

<u>Ölkörper</u>

Als Ölkörper kommen beispielsweise Guerbetalkohole auf Basis von Fettalkoholen mit 6 bis 18, vorzugsweise 8 bis 10 Kohlenstoffatomen, Ester von linearen $C_6$-$C_{22}$-Fettsäuren mit linearen oder verzweigten $C_6$-$C_{22}$-Fettalkoholen bzw. Ester von verzweigten $C_6$-$C_{13}$-Carbonsäuren mit linearen oder verzweigten $C_6$-$C_{22}$-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, I-sostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen $C_6$-$C_{22}$-Fettsäuren mit verzweigten Alkoholen, insbesondere 2-Ethylhexanol, Ester von $C_{18}$-$C_{38}$-Alkylhydroxycarbonsäuren mit linearen oder verzweigten $C_6$-$C_{22}$-Fettalkoholen (vgl. **DE 19756377 A1**), insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis $C_6$-$C_{10}$-Fettsäuren, flüssige Mono-/Di-/Triglyceridmischungen auf Basis von $C_6$-$C_{18}$-Fettsäuren, Ester von $C_6$-$C_{22}$-Fettalkoholen und/oder Guerbetalkoholen mit aromatischen Carbonsäuren, insbesondere Benzoesäure, Ester von $C_2$-$C_{12}$-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte $C_6$-$C_{22}$-Fettalkoholcarbonate, wie z.B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten $C_6$-$C_{22}$-Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol® OE), Ringöffnungsprodukte von epoxidierten Fettsäureestern mit Polyolen, Siliconöle (Cydomethicone, Siliciummethicontypen u.a.) und/oder aliphatische bzw. naphthenische Kohlenwasserstoffe, wie z.B. wie Squalan, Squalen oder Dialkylcyclohexane in Betracht.

<u>Emulgatoren</u>

Als Emulgatoren kommen beispielsweise nichtionogene Tenside aus mindestens einer der folgenden Gruppen in Frage:

- Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;

- Alkyl- und/oder Alkenyloligoglykoside mit 8 bis 22 Kohlenstoffatomen im Alk(en)ylrest und deren ethoxylierte Analoga;
- Anlagerungsprodukte von 1 bis 15 Mol Ethylenoxid an Ricinusöl und/oder gehärtetes Ricinusöl;
- Anlagerungsprodukte von 15 bis 60 Mol Ethylenoxid an Ricinusöl und/oder gehärtetes Ricinusöl;
- Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Mischester aus Pentaerythrit, Fettsäuren, Citronensäure und Fettalkohol gemäß DE **1165574 PS** und/oder Mischester von Fettsäuren mit 6 bis 22 Kohlenstoffatomen, Methylglucose und Polyolen, vorzugsweise Glycerin oder Polyglycerin.
- Mono-, Di- und Trialkylphosphate sowie Mono-, Di- und/oder Tri-PEG-alkylphosphate und deren Salze;
- Wollwachsalkohole; > Polysiloxan-Polyalkyl-Polyether-Copolymere bzw. entsprechende Derivate;
- Block-Copolymere z.B. Polyethylenglycol-30 Dipolyhydroxystearate;
- Polymeremulgatoren, z.B. Pemulen-Typen (TR-1,TR-2) von Goodrich;
- Polyalkylenglycole sowie
- Glycerincarbonat.

➤ <u>Ethylenoxidanlagerungsprodukte</u>

Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. $C_{12/18}$-Fettsäuremono- und -diester von Anlagerungsprodukten von Ethylenoxid an Glycerin sind aus **DE 2024051 PS** als Rückfettungsmittel für kosmetische Zubereitungen bekannt.

➤ <u>Alkyl- und/oder Alkenyloligoglykoside</u>

Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohfenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

➤ <u>Partialglyceride</u>

Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Unolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

➤ <u>Sorbitanester</u>

Als Sorbitanester kommen Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

➤ Polyglycerinester

Typische Beispiele für geeignete Polyglycerinester sind Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

➤ Anionische Emulgatoren

Typische anionische Emulgatoren sind aliphatische Fettsäuren mit 12 bis 22 Kohlenstoffatomen, wie beispielsweise Palmitinsäure, Stearinsäure oder Behensäure, sowie Dicarbonsäuren mit 12 bis 22 Kohlenstoffatomen, wie beispielsweise Azelainsäure oder Sebacinsäure.

➤ Amphothere und kationische Emulgatoren

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylatund eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethyl-ammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer $C_{8/18}$-Alkyl- oder Acylgruppe im Molekül mindestens eine freie Aminogruppe und mindestens eine -COOH- oder -$SO_3$H-Gruppe enthalten und zur Ausbildung innerer Salze befähigt sind. Beispiele für geeignete ampholytische Tenside sind N-Alkylglycine, N-Alkylpropion-säuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils wa 8 bis 18 C-Atomen in der Alkylgruppe.. Besonders bevorzugte ampholytische Tenside sind das N-Kokosalkylaminopropionat, das Kokosacylaminoethylaminopropionat und das $C_{12/18}$-Acylsarcosin. Schließlich kommen auch Kationtenside als Emulgatoren in Betracht, wobei solche vom Typ der Esterquats, vorzugsweise methylquaternierte Difettsäuretriethanolaminester-Salze, besonders bevorzugt sind.

Fette und Wachse

Typische Beispiele für Fette sind Glyceride, d.h. feste oder flüssige pflanzliche oder tierische Produkte, die im wesentlichen aus gemischten Glycerinestern höherer Fettsäuren bestehen, als Wachse kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC). Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

Perlglanzwachse

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Konsistenzgener und Verdickungsmittel

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und -diester von Fettsäuren, Polyacrylate, (z.B. Carbopole® und Pemulen-Typen von Goodrich; Synthalene® von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon. Als besonders wirkungsvoll haben sich auch Bentonite, wie z.B. Betone® Gel VS-5PC (Rheox) erwiesen, bei dem es sich um eine Mischung aus Cydopentasiloxan, Disteardimonium Hectorit und Propylencarbonat handelt. Weiter in Frage kommen Tenside, wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Überfettungsmittel

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Stabilisatoren

Als Stabilisatoren können Metallsalze von Fettsäuren, wie z.B. Magnesium-, Aluminiumund/oder Zinkstearat bzw. -ricinoleat eingesetzt werden.

Polymere

Geeignete kationische Polymere sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium Hydroxypropyl Hydrolyzed Collagen (Lamequat®L/Grünau), quatemierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyl-diallylammoniumchlorid (Merquat® 550/Chemviron), Polyaminopolyamide, wie z.B. beschrieben in der **FR 2252840 A** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als anionische, zwitterionische, amphotere und nichtionische Polymere kommen beispielsweise Vinylacetat/ Crotonsäure-Copolymere, Vinylpyrrolidon/Vinylacrylat-Copolymere, Vinylacetat/Butylmaleat/ Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchtorid/ Acrylat-Copolymere, Octylacrylamid/Methylmeth-acrylat/tert.Butylaminoethylmethacrylat/2-Hydroxypropylmethacrylat-Copolymere, Polyvinylpyrrolidon, Vinylpyrrolidon/ Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage. Weitere geeignete Polymere und Verdickungsmittel sind in **Cosm.Toil. 108, 95 (1993)** aufgeführt.

Siliconverbindungen

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silikaten handelt. Eine detaillierte Übersicht über geeignete flüchtige Silicone findet sich zudem von Todd et al. in **Cosm.Toil. 91, 27 (1976).**

UV-Lichtschutzfilter und Antioxidantien

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:

- 3-Benzylidencampher bzw. 3-Benzylidennorcampher und dessen Derivate, z.B. 3-(4-Methylbenzyliden)campher wie in der **EP 0693471 B1** beschrieben;
- 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
- Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
- Ester der Salicylsäure, vorzugsweise Salicylsäure-2-ethylhexylester, Saticylsäure-4-isopropylbenzylester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester; > Triazinderivate, wie z.B. 2,4,6-Trianilino-(p-carbo-2'-ethyl-1'-hexyloxy)-1,3,5-triazin und Octyl Triazon, wie in der **EP 0818450 A1** beschrieben oder Dioctyl Butamido Triazone (Uvasorb® HEB);
- Propan-1,3-dione, wie z.B. 1-(4-tert.Butylphenyl)-3-(4'methoxyphenyl)propan-1,3-dion;
- Ketotricyclo(5.2.1.0)decan-Derivate, wie in der **EP 0694521 B1** beschrieben.

Als wasserlösliche Substanzen kommen in Frage:

- 2-Phenylbenzimidazol-5-sulfonsäure und deren Alkali-, Erdalkali-, Ammonium-, Alkylammonium-, Alkanolammonium- und Glucammoniumsalze;
- Sulfonsäurederivate von Benzophenonen, vorzugsweise 2-Hydroxy-4-methoxybenzophenon-5-sulfonsäure und ihre Salze;
- Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'methoxydibenzoylmethan (Parsol® 1789), 1-Phenyl-3-(4'-isopropylphenyl)-propan-1,3-dion sowie Enaminverbindungen, wie beschrieben in der **DE 19712033 A1** (BASF). Die UV-A und UV-B-Filter können selbstverständlich auch in Mischungen eingesetzt werden. Besonders günstige Kombinationen bestehen aus den Derivate des Benzoylmethans,, z.B. 4-tert.-Butyl-4'-methoxydibenzoylmethan (Parsol® 1789) und 2-Cyano-3,3-phenylzimtsäure-2-ethylhexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden deartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen für diesen Zweck auch unlösliche Licht schutzpigmente, nämlich feindisperse Metalloxide bzw. Salze in Frage. Beispiele für geeignete Metalloxide sind insbesondere Zinkoxid

und Titandioxid und daneben Oxide des Eisens, Zirkoniums, Siliciums, Mangans, Aluminiums und Cers sowie deren Gemische. Als Salze können Silikate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in **SÖFW-Journal 122, 543 (1996)** sowie **Parf.Kosm. 3, 11 (1999)** zu entnehmen.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D, L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis μmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, $ZnSO_4$) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Biogene Wirkstoffe

Unter biogenen Wirkstoffen sind beispielsweise Tocopherol, Tocopherolacetat, Tocopherolpalmitat, Ascorbinsäure, (Desoxy)Ribonudeinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z. B. Prunusextrakt, Bambaranussextrakt und Vitaminkomplexe zu verstehen.

Deodorantien und keimhemmende Mittel

Kosmetische Deodorantien (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.

➤ Keimhemmende Mittel

Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)harnstoff, 2,4,4'-Trichlor-2'-hydroxy-diphenylether (Triclosan), 4-Chlor-3,5-dimethyl-phenol, 2,2'-Methylen-bis(6-brom-4-chlorphenol), 3-Methyl-4-(1-methylethyl)-phenol, 2-Benzyl-4-chlorphenol, 3-(4-Chlorphenoxy)-1,2-propandiol, 3-Iod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

➤ Enzyminhibitoren

Als Enzyminhibitoren sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester, sowie Zinkglycinat.

➤ Geruchsabsorber

Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcydohexylacetat, Linalylacetat, Phenylethylacetat, Unalylbenzoat, Benzylformiat, Allylcydohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellat, Citronellyloxyacetaldehyd, Cydamenaldehyd, Hydroxydtronellal, Lilial und Bourgeonal, zu den Ketonen z.B. die Jonone und Methylcedrylketon, zu den Alkoholen Anethol, Citronellol, Eugenol, Isoeugenol, Geraniol, Linatool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z. B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, $\alpha$-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, $\beta$-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

➤ Antitranspirantien

Antitranspirantien (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:

- ➤ adstringierende Wirkstoffe,
- ➤ Ölkomponenten,
- ➤ nichtionische Emulgatoren,
- ➤ Coemulgatoren,
- ➤ Konsistenzgeber,
- ➤ Hilfsstoffe wie z. B. Verdicker oder Komplexierungsmittel und/oder
- ➤ nichtwässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirko-nium-tetrachlorohydrat, Aluminium-Zirkonium-pentachlorohydrat und deren Komplexverbindungen z. B. mit Aminosäuren wie Glycin. Daneben können in Antitranspirantien übliche öllösliche und wasserlösliche Hilfsmittel in geringeren Mengen enthalten sein. Solche öllöslichen Hilfsmittel können z.B. sein:

- entzündungshemmende, hautschützende oder wohlriechende ätherische Öle,
- synthetische hautschützende Wirkstoffe und/oder
- öllösliche Parfümöle.

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

Filmbildner

Gebräuchliche Filmbildner sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

Antischuppenwirkstoffe

Als Antischuppenwirkstoffe kommen Piroctone Olamin (1-Hydroxy-4-methyl-6-(2,4,4-trimythylpentyl)-2-(1H)-pyridinonmonoethanolaminsalz), Baypival® (Climbazole), Ketoconazol®, (4-Acetyl-1-{-4-[2-(2.4-dichlorphenyl) r-2-(1H-imidazol-1-ylmethyl)-1,3-dioxylan-c-4-ylmethoxyphenyl}piperazin, Ketoconazol, Elubiol, Selendisulfid, Schwefel kolloidal, Schwefelpolyehtylenglykolsorbitanmonooleat, Schwefelrizinolpolyehtoxylat, Schwfel-teer Destillate, Salicylsäure (bzw. in Kombination mit Hexachlorophen), Undexylensäure Monoethanolamid Sulfosuccinat Na-Salz, Lamepon® UD (Protein-Undecylensäurekondensat), Zinkpyrithion, Aluminiumpyrithion und Magnesiumpyrithion / Dipyrithion-Magnesiumsulfat in Frage.

Quellmittel

Als Quellmittel für wässrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R. Lochhead in **Cosm.Toil. 108, 95 (1993)** entnommen werden.

Insekten-Repellentien

Als Insekten-Repellentien kommen N,N-Diethyl-m-toluamid, 1,2-Pentandiol oder Ethyl Butylacetylaminopropionate in Frage

Selbstbräuner und Depigmentierungsmittel

Als Selbstbräuner eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

Hydrotrope

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxyrgruppen. Die Polyole können noch weitere funktionelle Gruppen, insbesondere Aminogruppen, enthalten bzw. mit Stickstoff modifiziert sein. Typische Beispiele sind

- Glycerin;
- Alkylenglycole, wie beispielsweise Ethylenglycol, Diethylenglycol, Propylenglycol, Butylenglycol, Hexylenglycol

sowie Polyethylenglycole mit einem durchschnittlichen Molekulargewicht von 100 bis 1.000 Dalton;

- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

Konservierungsmittel

Als Konservierungsmittel eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

Parfümöle und Aromen

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, $\alpha$-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, $\alpha$-Hexylzimtaldehyd, Geraniol, Benzylaceton, Cydamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, $\beta$-Damascone, Geraniumöl Bourbon, Cydohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.

Farbstoffe

Als Farbstoffe können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Beispiele sind Kochenillerot A (C.I. 16255), Patentblau V (C.I.42051), Indigotin (C.I.73015), Chlorophyllin (C.I.75810), Chinolingelb (C.I.47005), Titandioxid (C.I.77891), Indanthrenblau RS (C.I. 69800) und Krapplack (C.I.58000). Als Lumineszenzfarbstoff kann auch Luminol enthalten sein. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-%-bezogen auf die Mittel - betragen. Die Herstellung der Mittel kann durch übliche Kalt - oder Heißprozesse erfolgen; vorzugsweise arbeitet

man nach der Phaseninversionstemperatur-Methode.

**Beispiele**

**Herstellbeispiel 1.** In einem 2-I-Dreihalskolben mit Rührer, Destillationsaufsatz und Vakuumanschluss wurden 406 g (2,82 Mol) Caprylsäure, 206 g (1,41 Mol) Adipinsäure und 0,8 g Natriumhypophosphit vorgelegt und auf 70 °C erwärmt. Anschließend wurde der Druck auf 35 mbar abgesenkt und portionsweise 420 g (2,82 Mol) Triethanolamin zugegeben. Die Temperatur wurde schrittweise bis auf 165 °C angehoben, der Druck bis auf 5 mbar reduziert, bis die Wasserabscheidung zum Stillstand gekommen und die Säurezahl auf einen Wert unter 5 mg KOH/g abgesunken war. 871 g (2,72 Mol) des auf diesem Wege hergestellten Esters wurde in einen zweiten Kolben überführt und bei 50 °C in 211 g Isopropylalkohol gelöst. Anschließend wurde unter Rühren portionsweise 324 g (2,57 Mol) Dimethylsulfat zugegeben und die Temperatur dabei über 4 h bei 70 °C gehalten.

**Herstellbeispiel 2.** Analog Beispiel 1 wurden 424 g (2,73 Mol) einer technischen 1:1-Mischung aus Capryl- und Caprinsäure, 200 g (1,37 Mol) Adipinsäure, 0,7 g Natriumhypophosphit und 408 g (2,74 Mol) Triethanolamin umgesetzt. Anschließend wurden 871 g (2,63 Mol) des resultierenden Esters in 132 g Isopropylalkohol gelöst und mit 315 g (2,5 Mol) Dimethylsulfat quaterniert.

**Herstellbeispiel 3.** Analog Beispiel 1 wurden 292 g (1,70 Mol) Caprinsäure, 124 g (0,85 Mol) Adipinsäure, 0,5 g Natriumhypophosphit und 253 g (1,70 Mol) Triethanolamin umgesetzt. Anschließend wurden 582 g (1,63 Mol) des resultierenden Esters in 137 g Isopropylalkohol gelöst und mit 194 g (1,54 Mol) Dimethylsulfat quaterniert.

**Vergleichsbeispiel V1.** Analog Beispiel 1 wurden 530 g (3,5 Mol) einer technischen 1:1-Mischung aus Capryl- und Caprinsäure, 0,7 g Natriumhypophosphit und 408 g (2,74 Mol) Triethanolamin umgesetzt. Anschließend wurden 871 g (2,63 Mol) des resultierenden Esters in 132 g Isopropylalkohol gelöst und mit 315 g (2,5 Mol) Dimethylsulfat quaterniert.

**Mikrobizide Wirksamkeit**. Die mikrobizide Wirksamkeit der erfindungsgemäß verwendeten quaternären Carbonsäurealkanolaminestersalze wurde gegenüber folgenden ausgewählten Testkeimen bestimmt:

| | | | |
|---|---|---|---|
| (a) | Staphylococcus aureus ATCC 6538: | (f) | Enterococcus faecium |
| (b) | Pseudomonas aruginosa ATCC 15442 | (g) | Mycobakterium smegmatis |
| (c) | Candida albicans ATCC 10231 | (h) | Aspergillus niger |
| (d) | Salmonella enteritidis | (i) | Microsporum gypseum |
| (e) | Escherichia coli | (j) | Trichophyton mentagrophytes |

Die Wirksamkeit der zu untersuchenden Verbindung wurde mit Hilfe des Suspensionstests in Anlehnung an die Richtlinien der Deutschen Gesellschaft für Hygiene und Mikrobiologie (DGHM) ermittelt. Unter Verwendung von Wasser einer Härte von 17°dH sowie gegebenenfalls von Isopropylalkohol wurden Testlösungen hergestellt, die die in den Tabellen angegebenen Mengen an Esterquats von 50, 100, 200 und 400 mg/l enthielten.

Bei Raumtemperatur wurden jeweils 0,1 ml Testkeimsuspension in Reagenzgläser mit jeweils 10 ml der Testlösungen pipettiert und vermischt. Nach unterschiedlichen Einwirkzeiten von 5, 15 und 60 Minuten wurden den Reagenzgläsern mit Hilfe einer Pipette 1 ml Material entnommen, und in 9 ml Inaktivatorlösung (Inaktivatorsubstanzen: 3,0 % Tween, 0,3 % Lecithin, 0,1 % Histidin) übertragen. Nach mindestens 5 und längstens 30 Minuten Inaktivierung erfolgte die Bestimmung der Lebendkeimzahl durch Ausplattierung auf Caseinpepton-Sojabohnenmehlpepton(CaSo) -Agarplatten. Unter Verwendung eines Spiralplaters wurde direkt der Inaktivierungsansatz auf CaSo-Agar ausplattiert. Die Proben wurden 24 bis 72 Stunden bei 37 °C bebrütet und anschließend makroskopisch auf Wachstum beurteilt und auf diesem Weg die Abtötungszeit oder der Restkeimgehalt ermittelt. Dabei wird die Keimzahl einer unbelasteten Wasserkontrolle in das Verhältnis zur Keimzahl der inaktivierten Testlösung unter Berücksichtigung der Verdünnung gestellt. In der nachfolgenden Tabellen 1 bis 10 sind die logarithmischen Reduktionsfaktoren angegeben, d.h. die Zehnerpotenzreduktion. Dabei gilt, je höher der Wert ist, desto besser ist die mikrobizide Wirkung. Die Angabe < bedeutet, dass der logarithmische Reduktionsfaktor unter dem angegebenen Wert bzw. bei der Angabe > über dem angegebenen Wert liegt. Die Abkürzung AKW steht für Anwendungskonzentration. Man erkennt, dass mit den erfindungsgemäß zu verwendenden Esterquats gegenüber denen des nächstliegenden Stands der Technik bei geringeren Einsatzmengen und niedrigeren Einwirkzeiten höhere Reduktionsraten erzielt werden.

**Tabelle 1**
**Staphylococcus aureus**

| Esterquat | Dauer [min] | 50 ppm | 100 ppm | 200 ppm | 400 ppm |
|---|---|---|---|---|---|
| H2 | 5 | 2,0 | 2,3 | 3,3 | > 5,6 |
| | 15 | 3,3 | 3,5 | 4,0 | > 5,6 |
| | 60 | 4,0 | > 5,6 | > 5,6 | > 5,6 |
| V1 | 5 | < 1,3 | < 1,3 | < 1,3 | < 1,3 |
| | 15 | < 1,7 | < 1,7 | < 1,7 | < 1,7 |
| | 60 | < 1,3 | < 1,3 | 1,9 | 3,5 |

**Tabelle 2**
**Pseudomonas aeruginosa**

| Esterquat | Dauer [min] | 50 ppm | 100 ppm | 200 ppm | 400 ppm |
|---|---|---|---|---|---|
| H2 | 5 | 2,2 | 4,0 | > 5,6 | > 5,6 |
| | 15 | > 5,6 | > 5,6 | > 5,6 | > 5,6 |
| | 60 | > 5,6 | > 5,6 | > 5,6 | > 5,6 |
| V1 | 5 | < 1,4 | < 1,7 | 2,2 | 4,0 |
| | 15 | < 1,7 | 4,0 | 4,4 | > 5,6 |
| | 60 | 4,0 | 4,4 | > 5,6 | > 5,6 |

**Tabelle 3**
**Candida albicans**

| Esterquat | Dauer [min] | 50 ppm | 100 ppm | 200 ppm | 400 ppm |
|---|---|---|---|---|---|
| H2 | 5 | > 5,6 | > 5,6 | > 5,6 | > 5,6 |
| | 15 | > 5,6 | > 5,6 | > 5,6 | > 5,6 |
| | 60 | > 5,6 | > 5,6 | > 5,6 | > 5,6 |
| V1 | 5 | > 3,4 | > 5,6 | > 5,6 | > 5,6 |
| | 15 | > 5,6 | > 5,6 | > 5,6 | > 5,6 |
| | 60 | > 5,6 | > 5,6 | > 5,6 | > 5,6 |

**Tabelle 4**
**Salmonella Enteridis**

| Esterquat | Dauer [min] | 50 ppm | 100 ppm | 200 ppm | 400 ppm |
|---|---|---|---|---|---|
| H2 | 5 | < 1,8 | 4,0 | 4,9 | > 5,8 |
| | 15 | 3,3 | 5,1 | > 5,8 | > 5,8 |
| | 60 | 3,7 | > 5,8 | > 5,8 | > 5,8 |
| V1 | 5 | < 1,8 | < 1,8 | 3,6 | 4,9 |
| | 15 | 2,2 | 3,5 | 5,1 | > 5,8 |
| | 60 | 3,5 | > 5,8 | > 5,8 | > 5,8 |

**Tabelle 5**
**Escherichia Coli**

| Esterquat | Dauer [min] | 50 ppm | 100 ppm | 200 ppm | 400 ppm |
|---|---|---|---|---|---|
| H2 | 5 | 4,1 | > 5,8 | > 5,8 | > 5,8 |
| | 15 | > 5,8 | > 5,8 | > 5,8 | > 5,8 |
| | 60 | > 5,8 | > 5,8 | > 5,8 | > 5,8 |
| V1 | 5 | 3,5 | 4,2 | > 5,8 | > 5,8 |
| | 15 | 4,0 | > 5,8 | > 5,8 | > 5,8 |
| | 60 | > 5,8 | > 5,8 | > 5,8 | > 5,8 |

**Tabelle 6**
**Enterococcus faecium**

| Esterquat | Dauer [min] | 50 ppm | 100 ppm | 200 ppm | 400 ppm |
|---|---|---|---|---|---|
| H2 | 5 | > 6,3 | > 6,3 | > 6,3 | > 6,3 |
| | 15 | > 5,8 | > 5,8 | > 5,8 | > 5,8 |
| | 60 | > 5,8 | > 5,8 | > 5,8 | > 5,8 |
| V1 | 5 | > 5,8 | > 5,8 | > 5,8 | > 5,8 |
| | 15 | > 5,8 | > 5,8 | > 5,8 | > 5,8 |
| | 60 | > 5,8 | > 5,8 | > 5,8 | > 5,8 |

**Tabelle 7**
**Mycobakterium smegmatis**

| Esterquat | Dauer [min] | 50 ppm | 100 ppm | 200 ppm | 400 ppm |
|---|---|---|---|---|---|
| H2 | 5 | < 1,4 | < 1,4 | < 1,4 | < 1,4 |
| | 15 | < 1,5 | < 1,5 | < 1,5 | < 1,5 |
| | 60 | < 1,7 | < 1,7 | < 1,7 | < 1,7 |
| V1 | 5 | < 1,4 | < 1,4 | < 1,4 | < 1,4 |
| | 15 | < 1,4 | < 1,4 | < 1,4 | < 1,4 |
| | 60 | < 1,5 | < 1,5 | < 1,5 | < 1,5 |

**Tabelle 8**
**Aspergillus niger**

| Esterquat | Dauer [min] | 50 ppm | 100 ppm | 200 ppm | 400 ppm |
|---|---|---|---|---|---|
| H2 | 5 | < 0,5 | < 0,5 | < 0,5 | < 0,5 |
| | 15 | 0,2 | 0,2 | 0,2 | 0,3 |
| | 60 | 0,2 | 0,5 | 0,4 | 0,5 |
| V1 | 5 | < 0,5 | < 0,5 | < 0,5 | < 0,5 |
| | 15 | < 0,5 | < 0,5 | < 0,5 | < 0,5 |
| | 60 | 0,2 | 0,2 | 0,2 | 0,2 |

**Tabelle 9**
**Mycosporum gypseum**

| Esterquat | Dauer [min] | 50 ppm | 100 ppm | 200 ppm | 400 ppm |
|---|---|---|---|---|---|
| H2 | 5 | > 3,5 | > 3,5 | > 3,5 | > 3,5 |
| | 15 | > 3,5 | > 3,5 | > 3,5 | > 3,5 |
| | 60 | > 3,6 | > 3,6 | > 3,7 | > 3,7 |
| V1 | 5 | > 3,5 | > 3,5 | > 3,5 | > 3,5 |
| | 15 | > 3,5 | > 3,5 | > 3,5 | > 3,5 |
| | 60 | > 3,5 | > 3,5 | > 3,5 | > 3,5 |

**Tabelle 10**
**Trychophyton mentagrophytes**

| Esterquat | Dauer [min] | 50 ppm | 100 ppm | 200 ppm | 400 ppm |
|---|---|---|---|---|---|
| H2 | 5 | 0,3 | 0,4 | 0,3 | 0,4 |
| | 15 | 1,0 | 1,0 | 0,9 | 0,9 |
| | 60 | 2,0 | 2,0 | 2,2 | 2,3 |
| V1 | 5 | 0,3 | 0,3 | 0,3 | 0,3 |
| | 15 | 1,0 | 1,0 | 1,0 | 1,0 |
| | 60 | 1,5 | 1,5 | 1,5 | 1,5 |

## Patentansprüche

1. Verwendung von Esterquats als Biozide, **dadurch gekennzeichnet, dass** sich die Acylkomponente der Esterquats von Gemischen von

   (a) Monocarbonsäuren und
   (b) Dicarbonsäuren

   ableitet.

2. Verwendung nach Anspruch 1, **dadurch gekennzeichnet, dass** man Esterquats verwendet, deren Acylkomponente (a) sich von Monocarbonsäuren der Formel **(I)** ableiten,

   $$R^1CO\text{-}OH \qquad (I)$$

   in der $R^1CO$ für einen linearen oder verzweigten, gesättigten oder ungesättigten, gegebenenfalls hydroxysubstituierten Acylrest mit 6 bis 22 Kohlenstoffatomen steht.

3. Verwendung nach den Ansprüchen 1 und/oder 2, **dadurch gekennzeichnet, dass** man Esterquats verwendet, deren Acylkomponente (a) sich von Monocarbonsäuren der Formel **(I)** ableitet, in der $R^1CO$ für einen linearen, gesättigten Acylrest mit 6 bis 12 Kohlenstoffatomen steht.

4. Verwendung nach mindestens einem der Ansprüche 1 bis 3, **dadurch gekennzeichnet, dass** man Esterquats verwendet, deren Acylkomponente (b) sich von Dicarbonsäuren der Formel **(II)** ableitet,

   $$HOOC(CH_2)_nCOOH \qquad (II)$$

   in der n für Zahlen von 1 bis 10 steht.

5. Verwendung nach mindestens einem der Ansprüche 1 bis 4, **dadurch gekennzeichnet, dass** man Esterquats verwendet, deren Acylkomponente (b) sich von der Adipinsäure ableitet.

6. Verwendung nach mindestens einem der Ansprüche 1 bis 5, **dadurch gekennzeichnet, dass** man Esterquats verwendet, deren Acylkomponente (a) sich von Monocarbonsäuren der Formel **(I)** ableitet, in der $R^1CO$ für einen linearen, gesättigten Acylrest mit 6 bis 22 Kohlenstoffatomen und deren Acylkomponente (b) von der Adipinsäure ableitet.

7. Verwendung nach mindestens einem der Ansprüche 1 bis 6, **dadurch gekennzeichnet, dass** man Esterquats

verwendet, bei denen das molare Verhältnis der Komponenten (a) und (b) im Bereich von 1 : 99 bis 99 : 1 liegt.

**8.** Verwendung nach mindestens einem der Ansprüche 1 bis 7, **dadurch gekennzeichnet, dass** man Esterquats der Formel **(III)** einsetzt,

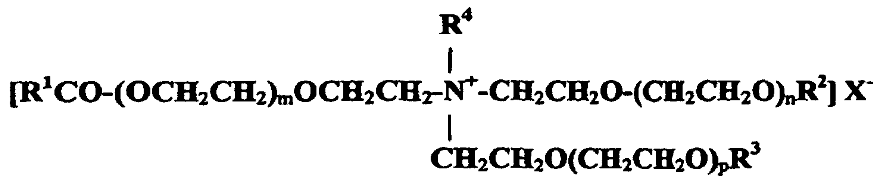

**(III)**

in der $R^1CO$ für Mischungen von Acylresten von Mono- und Dicarbonsäuren, $R^2$ und $R^3$ unabhängig voneinander für Wasserstoff oder $R^1CO$, $R^4$ für einen Alkylrest mit 1 bis 4 Kohlenstoffatomen oder eine $(CH_2CH_2O)_qH$-Gruppe, m, n und p in Summe für 0 oder Zahlen von 1 bis 12, q für Zahlen von 1 bis 12 und X für Halogenid, Alkylsulfat oder Alkylphosphat steht.

**9.** Verwendung nach mindestens einem der Ansprüche 1 bis 7, **dadurch gekennzeichnet, dass** man Esterquats der Formel **(IV)** einsetzt,

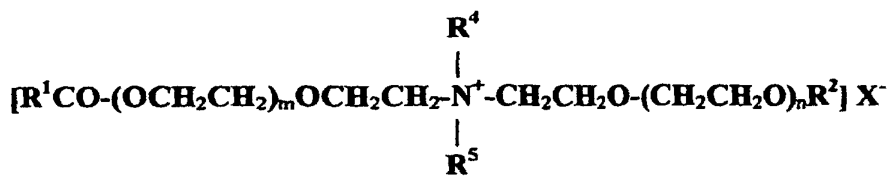

**(IV)**

in der $R^1CO$ für Mischungen von Acylresten von Mono- und Dicarbonsäuren, $R^2$ für Wasserstoff oder $R^1CO$, $R^4$ und $R^5$ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

**10.** Verwendung nach mindestens einem der Ansprüche 1 bis 7, **dadurch gekennzeichnet, dass** man Esterquats der Formel **(V)** einsetzt,

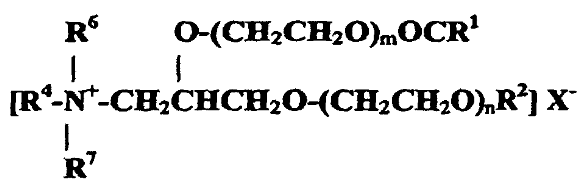

**(V)**

in der $R^1CO$ für Mischungen von Acylresten von Mono- und Dicarbonsäuren, $R^2$ für Wasserstoff oder $R^1CO$, $R^4$, $R^6$ und $R^7$ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

**Claims**

**1.** The use of esterquats as biocides, **characterized in that** the acyl component of the esterquats is derived from mixtures of

(a) monocarboxylic acids and
(b) dicarboxylic acids.

2. The use claimed in claim 1, **characterized in that** esterquats of which the acyl component (a) is derived from monocarboxylic acids corresponding to formula **(I):**

$$R^1CO\text{-}OH \qquad (I)$$

in which $R^1CO$ is a linear or branched, saturated or unsaturated, optionally hydroxysubstituted acyl group containing 6 to 22 carbon atoms, are used.

3. The use claimed in claims 1 and/or 2, **characterized in that** esterquats of which the acyl component (a) is derived from monocarboxylic acids corresponding to formula **(I),** in which $R^1CO$ is a linear, saturated acyl group containing 6 to 12 carbon atoms, are used.

4. The use claimed in at least one of claims 1 to 3, **characterized in that** estersquats of which the acyl component (b) is derived from dicarboxylic acids corresponding to formula **(II)**:

$$HOOC(CH_2)_nCOOH \qquad (II)$$

in which n is a number of 1 to 10,
are used.

5. The use claimed in at least one of claims 1 to 4, **characterized in that** estersquats of which the acyl component (b) is derived from adipic acid are used.

6. The use claimed in at least one of claims 1 to 5, **characterized in that** estersquats of which the acyl component (a) is derived from monocarboxylic acids corresponding to formula **(I)**, in which $R^1CO$ is a linear, saturated acyl group containing 6 to 22 carbon atoms, and of which the acyl component (b) is derived from adipic acid are used.

7. The use claimed in at least one of claims 1 to 6, **characterized in that** esterquats where the molar ratio of component (a) to component (b) is 1:99 to 99:1 are used.

8. The use claimed in at least one of claims 1 to 7, **characterized in that** esterquats corresponding to formula **(III):**

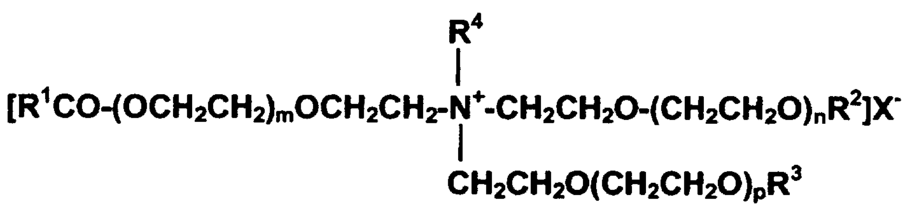

**(III)**

in which $R^1CO$ stands for mixtures of acyl groups of mono- and dicarboxylic acids, $R^2$ and $R^3$ independently of one another represent hydrogen or have the same meaning as $R^1CO$, $R^4$ is an alkyl group containing 1 to 4 carbon atoms or a $(CH_2CH_2O)_qH$ group, m, n and p together stand for 0 or numbers of 1 to 12, q is a number of 1 to 12 and X is halide, alkyl sulfate or alkyl phosphate,
are used.

9. The use claimed in at least one of claims 1 to 7, **characterized in that** esterquats corresponding to formula **(IV)**:

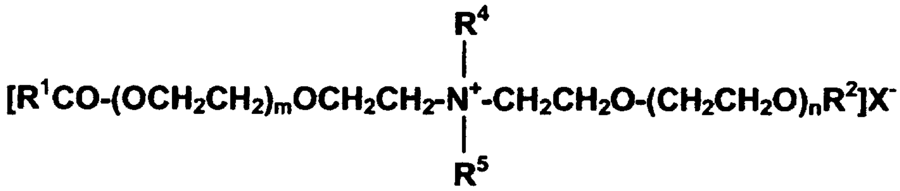 (IV)

in which $R^1CO$ stands for mixtures of acyl groups of mono- and dicarboxylic acids, $R^2$ is hydrogen or has the same meaning as $R^1CO$, $R^4$ and $R^5$ independently of one another are alkyl groups containing 1 to 4 carbon atoms, m and n together stand for 0 or numbers of 1 to 12 and X stands for halide, alkyl sulfate or alkyl phosphate, are used.

**10.** The use claimed in at least one of claims 1 to 7, **characterized in that** esterquats corresponding to formula **(V):**

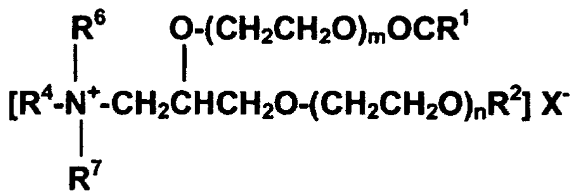 (V)

in which $R^1CO$ stands for mixtures of acyl groups of mono- and dicarboxylic acids, $R^2$ is hydrogen or has the same meaning as $R^1CO$, $R^4$, $R^6$ and $R^7$ independently of one another are alkyl groups containing 1 to 4 carbon atoms, m and n together stand for 0 or numbers of 1 to 12 and X stands for halide, alkyl sulfate or alkyl phosphate, are used.

**Revendications**

**1.** Utilisation d'ammonium quaternaires estérifiés en tant que biocides,
**caractérisée en ce que**
le composant acyle des d'ammonium quaternaires estérifiés dérive de mélanges

(a) d'acides monocarboxyliques et
(b) d'acides dicarboxyliques.

**2.** Utilisation selon la revendication 1,
**caractérisée en ce qu'**
on met en oeuvre des ammonium quaternaires estérifiés dont les composants acyle (a) sont dérivés d'acides monocarboxyliques correspondant à la formule (I)

$$R^1CO\text{-}OH \qquad (I)$$

dans laquelle $R^1CO$ représente un radical acyle linéaire ou ramifié, saturé ou insaturé, éventuellement hydroxy substitué, comportant de 6 à 22 atomes de carbone.

**3.** Utilisation selon les revendications 1 et/ou 2,
**caractérisée en ce qu'**
on met en oeuvre des d'ammonium quaternaires estérifiés dont le composant acyle (a) est dérivé d'acides monocarboxyliques correspondant à la formule (I), dans laquelle $R^1CO$ représente un radical acyle linéaire saturé comportant de 6 à 12 atomes de carbone.

4. Utilisation selon au moins une des revendications 1 à 3, **caractérisée en ce qu'** on met en oeuvre des d'ammonium quaternaires estérifiés dont le composant acyle (b) dérive d'acides dicarboxyliques correspondant à la formule (II)

$$HOOC(CH_2)_nCOOH \qquad (II)$$

dans laquelle n représente des nombres valant de 1 à 10.

5. Utilisation selon au moins une des revendications 1 à 4, **caractérisée en ce qu'** on met en oeuvre des d'ammonium quaternaires estérifiés dont le composant acyle (b) dérive de l'acide adipique.

6. Utilisation selon au moins une des revendications 1 à 5, **caractérisée en ce qu'** on met en oeuvre des ammonium quaternaires estérifiés dont le composant acyle (a) dérive d'acides monocarboxyliques correspondant à la formule (I), dans laquelle $R^1CO$ représente un radical acyle linéaire saturé comportant de 6 à 22 atomes de carbone et dont le composant acyle (b) dérive de l'acide adipique.

7. Utilisation selon au moins une des revendications 1 à 6, **caractérisée en ce qu'** on met en oeuvre des ammonium quaternaires estérifiés dans lesquels le rapport molaire entre les composants (a) et (b) se situe entre 1 : 99 et 99 : 1.

8. Utilisation selon au moins une des revendications 1 à 7, **caractérisée en ce qu'** on met en oeuvre des ammonium quaternaires estérifiés correspondant à la formule (III)

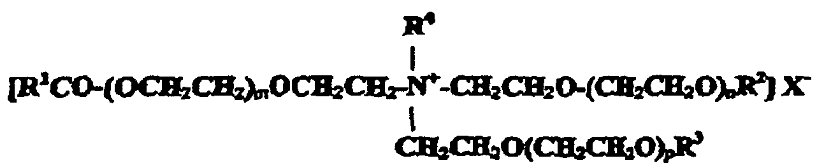

(III)

dans laquelle $R^1CO$ représente des mélanges de radicaux acyle d'acides monocarboxyliques et dicarboxyliques, $R^2$ et $R^3$ représentent, indépendamment l'un de l'autre, un atome d'hydrogène ou $R^1CO$, $R^4$ représente un radical alkyle comportant de 1 à 4 atomes de carbone ou un groupe $(CH_2CH_2O)_qH$, m, n et p ont pour somme 0 ou des nombres allant de 1 à 12, q représente des nombres allant de 1 à 12 et X représente un halogénure, un sulfate d'alkyle ou un phosphate d'alkyle.

9. Utilisation selon au moins une des revendications 1 à 7, **caractérisée en ce qu'** on met en oeuvre des ammonium quaternaires estérifiés correspondant à la formule (IV)

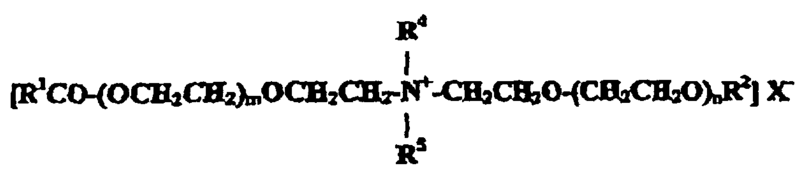

(IV)

dans laquelle $R^1CO$ représente des mélanges de radicaux acyle d'acides monocarboxyliques et dicarboxyliques, $R^2$ représente un atome d'hydrogène ou $R^1CO$, $R^4$ et $R^5$ représentent, indépendamment l'un de l'autre, un radical alkyle comportant de 1 à 4 atomes de carbone, m et n ont pour somme 0 ou des nombres allant de 1 à 12 et X

représente un halogénure, un sulfate d'alkyle ou un phosphate d'alkyle.

**10.** Utilisation selon au moins une des revendications 1 à 7,
**caractérisée en ce qu'**
on met en oeuvre des ammonium quaternaires estérifiés correspondant à la formule (V)

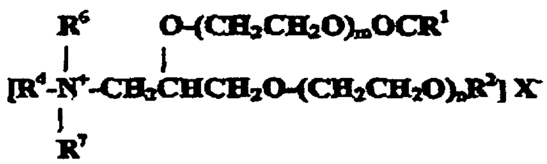

(V)

dans laquelle $R^1CO$ représente des mélanges de radicaux acyle d'acides monocarboxyliques et dicarboxyliques, $R^2$ représente un atome d'hydrogène ou $R^1CO$, $R^4$, $R^6$ et $R^7$ représentent, indépendamment l'un de l'autre, un radical alkyle comportant de 1 à 4 atomes de carbone, m et n ont pour somme 0 ou des nombres allant de 1 à 12 et X représente un halogénure, un sulfate d'alkyle ou un phosphate d'alkyle.